(19) Europäisches Patentamt
European Patent Office
Office européen des brevets

(11) **EP 4 293 684 B1**

(12) **EUROPEAN PATENT SPECIFICATION**

(45) Date of publication and mention of the grant of the patent:
**17.06.2026 Bulletin 2026/25**

(21) Application number: **22778823.9**

(22) Date of filing: **26.03.2022**

(51) International Patent Classification (IPC):
**G16H 50/30** (2018.01)   **A61B 5/02** (2006.01)

(52) Cooperative Patent Classification (CPC):
**G16H 40/67; A61B 5/02108; A61B 5/7267; A61B 5/7275; A61B 5/742; G16H 50/20; G16H 50/30; G16H 50/70;** A61B 5/02416; A61B 5/681

(86) International application number:
**PCT/CN2022/083229**

(87) International publication number:
**WO 2022/206641 (06.10.2022 Gazette 2022/40)**

(54) **HYPERTENSION RISK MEASUREMENT AND RELATED APPARATUS**

MESSUNG DES BLUTHOCHDRUCKRISIKOS UND ZUGEHÖRIGE VORRICHTUNG

MESURE DE RISQUE D'HYPERTENSION ET APPAREIL ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2021 CN 202110344922**

(43) Date of publication of application:
**20.12.2023 Bulletin 2023/51**

(73) Proprietor: **Huawei Technologies Co., Ltd.**
**Shenzhen, Guangdong 518129 (CN)**

(72) Inventors:
• **LI, Liangzhi**
 **Shenzhen, Guangdong 518129 (CN)**
• **LI, Luping**
 **Shenzhen, Guangdong 518129 (CN)**

• **CHEN, Maolin**
 **Shenzhen, Guangdong 518129 (CN)**

(74) Representative: **Maiwald GmbH**
**Engineering**
**Elisenhof**
**Elisenstrasse 3**
**80335 München (DE)**

(56) References cited:
WO-A1-2020/073012   WO-A1-2020/073012
CN-A- 106 343 976   CN-A- 107 028 603
CN-A- 107 438 399   CN-A- 107 847 156
CN-A- 107 863 156   CN-A- 109 767 836
CN-A- 110 291 555   CN-A- 111 970 965
CN-A- 112 420 195   US-A1- 2014 168 246
US-A1- 2018 075 209   US-A1- 2019 104 951
US-A1- 2020 320 335

**Description**

**TECHNICAL FIELD**

This application relates to the field of terminal technologies, and in particular, to a hypertension risk detection system, a related electronic device, and a computer storage medium comprising computer instructions to be run on the electronic device.

**BACKGROUND**

Blood pressure is pressure of blood circulation on an arterial wall of a body and is important data that reflects a cardiovascular ability of the human body. The blood pressure may include systolic blood pressure and diastolic blood pressure. The systolic blood pressure may show pressure in a blood vessel when a heart contracts or beats. The diastolic blood pressure may show pressure in the blood vessel when the heart relaxes between two times of beating. Excessively high systolic blood pressure and/or diastolic blood pressure may be diagnosed as hypertension. Most hypertensive patients may not be aware of their hypertension due to a lack of a warning sign or a symptom. Therefore, regular blood pressure detection helps a user understand a physical condition and reduces a risk caused by the hypertension.

Currently, some electronic devices (such as watches) may detect the blood pressure by detecting a photoplethysmography (photoplethysmography, PPG) signal of the user. The PPG signal includes a large amount of physiological information. Features such as a waveform, a periodicity and a peak value of the PPG signal are closely related to information such as a cardiovascular status, a degree of arteriosclerosis, and a heart rate. The electronic device may establish a model related to the PPG signal and the blood pressure, and perform hypertension risk detection by using a feature extracted from the PPG signal.

However, different users have different physical conditions. When using the foregoing heart rate detection method for the first time to detect a heart rate, the user needs to calibrate the foregoing model. In addition, a physical condition of a same user changes with time (where for example, vascular elasticity changes with time). When detecting a heart rate by using the foregoing heart rate detection method, the user needs to frequently calibrate the foregoing model. In the foregoing calibration method, the user usually needs to use a more professional hypertension risk detection device or go to hospital for detection to obtain blood pressure with high accuracy. A calibration process is cumbersome.

WO 2020/073012 A1 describes a wearable computing system used to collect user heart rate data which is labeled with a diagnosis. The heart rate data is used to train a machine learning model to predict when a user is experiencing an event associated with the label (e.g., high blood pressure).

**SUMMARY**

The invention is defined by the appended claims.

This application provides a hypertension risk detection system, a related electronic device, and a computer storage medium comprising computer instructions to be run on the electronic device, as defined by the appended claims. An electronic device stores a plurality of blood pressure models. The electronic device may obtain a PPG signal of a user, and select, from the plurality of blood pressure models by using the PPG signal, a blood pressure model that is adapted to the user. The electronic device may detect a degree of a hypertension risk of the user by using the selected blood pressure model. According to the foregoing method, impact of different physical conditions of different users and a change of a physical condition of a same user on a detection result can be reduced, and a calibration operation performed by the user on the electronic device when the user detects the degree of the hypertension risk can be simplified, thereby improving convenience of performing hypertension risk detection by the user.

This application provides a hypertension risk detection method. The method is: obtaining a first PPG signal of a first user, and selecting q blood pressure models from p blood pressure models for the first user. The p blood pressure models are obtained by training p groups of training samples. One of the p blood pressure models is obtained by training one of the p groups of training samples. The group of training samples includes features of a plurality of PPG signals of one user and a degree of a hypertension risk of the user that is detected when the PPG signal is collected. The plurality of obtained PPG signals of the group of training samples may be collected in a first time period. p is a positive integer. q is a positive integer less than or equal to p. A degree of a hypertension risk of the first user may be determined by using the q blood pressure models and the first PPG signal.

It can be learned from the foregoing method that, an electronic device may select, from the p blood pressure models, blood pressure models that are adapted to different users, to reduce impact of different physical conditions of different users on a detection result. In addition, because the physical condition of the user may change, the electronic device may periodically or irregularly update the blood pressure model for detecting the degree of the hypertension risk of the user. The electronic device may select one or more blood pressure models from the plurality of blood pressure models based on a currently collected PPG signal of the user. The one or more blood pressure models are most adapted to a user in a current physical condition. In this way, impact of a change of the physical condition of the user on a detection result can be reduced, thereby improving accuracy of detecting the degree of the hypertension risk of the user. In addition, according to the foregoing hypertension risk detection method, a calibration operation performed by the user on the electronic device when the user detects the degree of the hypertension risk of the user can be simplified, thereby improving convenience of performing hypertension risk detection by the user.

In some embodiments, when the PPG signal (for example, the first PPG signal) is obtained, preprocessing (for example, filtering) may be performed on the PPG signal. Further, feature extraction may be performed on the preprocessed PPG signal to obtain the feature of the PPG signal. For example, the feature of the PPG signal may include a mean of rate-to-rate intervals RRIs, a median of the RRIs, a minimum value of the RRIs, a coefficient of variation of rate-to-rate intervals CVRR, a standard deviation of normal-to-normal intervals SDNN, a main peak value of the PPG signal, a rise time interval of the PPG signal, a fall time interval of the PPG signal, and the like.

With reference to the first aspect, in some embodiments, the method for selecting the q blood pressure models from the p blood pressure models for the first user may be: obtaining a second PPG signal of the first user, and selecting the q blood pressure models from the p blood pressure models by using the second PPG signal. The q blood pressure models are obtained by training q groups of training samples in the p groups of training samples. Features of PPG signals in the q groups of training samples differ least from a feature of the second PPG signal in the p groups of training samples.

With reference to the first aspect, in some embodiments, the method for selecting the q blood pressure models from the p blood pressure models for the first user may be: obtaining physiological feature information of the first user, where the physiological feature information may include one or more of the following: an age, a gender, a height, and a weight, and selecting the q blood pressure models from the p blood pressure models by using the physiological feature information of the first user. The q blood pressure models are obtained by training q groups of training samples in the p groups of training samples. Physiological feature information of a user to which PPG signals of the q groups of training samples belong and the physiological feature information of the first user fall within a same value range.

Physiological feature information of a user to which a PPG signal of each of the p groups of training samples belongs corresponds to a respective value range. For example, a first value range is a gender of a female whose age is 20 to 30, height is 150 to 170 cm, and weight is 40 to 60 kg. If a gender of the first user is a female whose age is 20, height is 160 cm, and weight is 50 kg, the physiological feature information of the first user falls within the first value range. A training sample in which physiological feature information of a user to which a PPG signal belongs falls within the first value range may be selected from the p groups of training samples. A blood pressure model trained by using the selected training sample is a blood pressure model that is adapted to the first user.

With reference to the first aspect, in some embodiments, the method for selecting the q blood pressure models from the p blood pressure models for the first user may be: obtaining physiological feature information of the first user, where the physiological feature information includes one or more of the following: an age, a gender, a height, and a weight, selecting w blood pressure models from the p blood pressure models by using the physiological feature information of the first user, where the w blood pressure models are obtained by training w groups of training samples in the p groups of training samples, physiological feature information of a user to which PPG signals of the w groups of training samples belong and the physiological feature information of the first user fall within a same value range, and w is a positive integer less than or equal to p and greater than or equal to q, and obtaining a second PPG signal of the first user, and selecting the q blood pressure models from the w blood pressure models by using the second PPG signal. The q blood pressure models are obtained by training q groups of training samples in the w groups of training samples, and features of PPG signals of the q groups of training samples differ least from a feature of the second PPG signal in the w groups of training samples.

Different blood pressure models are adapted to people of different genders, different ages, different heights, and different weights. Usually, a higher age indicates a higher value of a hypertension risk. Blood pressure models that are adapted to users of a same age class may be similar. The blood pressure model that is adapted to the user is first selected based on physiological feature information, and then the blood pressure model is further selected based on a feature of a PPG signal, so that efficiency of selecting the blood pressure model that is adapted to the user can be improved.

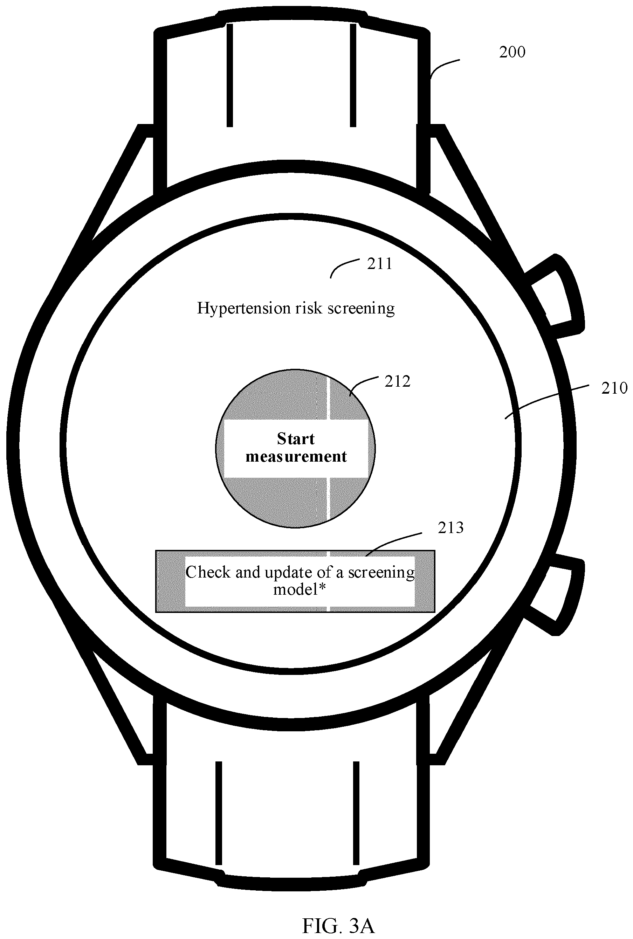

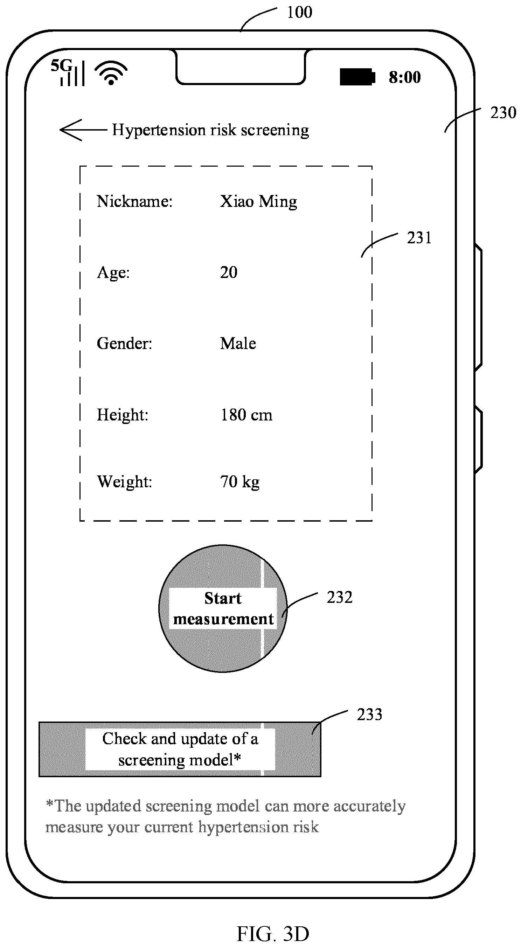

In some embodiments, the first PPG signal may be collected when the degree of the hypertension risk is detected. For example, in response to a user operation performed on a measurement start control 212 shown in FIG. 3A, or in response to a user operation performed on a measurement start control 232 shown in FIG. 3D, the electronic device may collect a PPG signal to obtain the first PPG signal. The second PPG signal may be collected when the blood pressure model that is adapted to the first user is selected. For example, when the blood pressure model is automatically updated periodically or irregularly, or in response to a user operation for updating the blood pressure model (for example, a user operation performed on a model update control 213 shown in FIG. 3A or a user operation performed on a model update control 233 shown in FIG. 3D), the electronic device may collect a PPG signal to obtain the second PPG signal.

The first PPG signal and the second PPG signal may be a same PPG signal. For example, the blood pressure model that is adapted to the first user is not determined, and in response to a user operation for measuring the degree of the hypertension risk (for example, the user operation performed on the measurement start control 212 shown in FIG. 3A or the user operation performed on the measurement start control 232 shown in FIG. 3D), the electronic device may collect a PPG signal. The PPG signal may be for selecting the blood pressure model that is adapted to the first user, and may also be for detecting the degree of the hypertension risk of the first user.

With reference to the first aspect, the plurality of obtained PPG signals of the group of training samples are collected in the first time period. A time length of the first time period may be, for example, one week or one month. In the first time period, a physical condition of a person usually does not change significantly. The plurality of PPG signals collected in the first time period may reflect a physical condition of the person in the first time period. The blood pressure model obtained through training by using the group of training samples can accurately detect a degree of a hypertension risk of a user in a corresponding physical condition.

With reference to the first aspect, a time interval between time for collecting the first PPG signal and time for collecting the second PPG signal does not exceed a time length of a second time period. The time length of the second time period may be, for example, one week or one month. The first PPG signal may be for determining the degree of the hypertension risk of the user. The second PPG signal may be for selecting the blood pressure model that is adapted to the user. The blood pressure model that is adapted to the user may be updated periodically or irregularly, or updated in response to the user operation for updating the blood pressure model. A time period between two consecutive updates of the blood pressure model is the second time period. In the time period between the two consecutive updates of the blood pressure model, a blood pressure model selected during a previous update of the blood pressure model may be used for detecting the degree of the hypertension risk of the user.

With reference to the first aspect, before the second PPG signal of the first user is obtained, a first user operation is received. The first user operation is a user operation indicating to select a blood pressure model.

With reference to the first aspect, a degree of a hypertension risk of a measured person that is detected when one PPG signal is collected may be a probability that systolic blood pressure of the measured person is higher than 140 mmHg when the PPG signal is collected. Alternatively, a degree of a hypertension risk of a measured person that is detected when one PPG signal is collected may be a probability that diastolic blood pressure of the measured person is higher than 90 mmHg when the PPG signal is collected.

With reference to the first aspect, in some embodiments, the p blood pressure models are included in a model library. The model library may further include more blood pressure models. Optionally, the model library may be updated. The updated model library may include more blood pressure models. An adapted blood pressure model may be selected for more users with different physical conditions by using the updated model library.

According to a second aspect, this application provides a hypertension risk detection system. The system may include a first electronic device and a second electronic device. The second electronic device may be configured to: select q blood pressure models from p blood pressure models for a first user, and send the q blood pressure models to the first electronic device. The p blood pressure models are obtained by training p groups of training samples, and one of the p blood pressure models is obtained by training one group of training samples. The group of training samples includes features of a plurality of PPG signals of one user and a degree of a hypertension risk of the user that is detected when the PPG signal is collected. The plurality of obtained PPG signals of the group of training samples are collected in a first time period. p is a positive integer, and q is a positive integer less than or equal to p. The first electronic device may be configured to: collect a first PPG signal of the first user, and determine a degree of a hypertension risk of the first user by using the q blood pressure models and the first PPG signal.

With reference to the second aspect, in some embodiments, the first electronic device may be a wearable device, for example, a watch or a band. The second electronic device may be an electronic device, for example, a mobile phone or a tablet computer. In some other embodiments, the first electronic device may be a wearable device, for example, a watch or a band. The second electronic device may be a cloud server. In some other embodiments, the first electronic device may be an electronic device, for example, a mobile phone or a tablet computer. The second electronic device may be a cloud server. Specific types of the first electronic device and the second electronic device are not limited in this embodiment of this application.

It can be learned from the foregoing system that the second electronic device may select, from a plurality of blood pressure models, a blood pressure model that is adapted to a user. The first electronic device may determine a degree of a hypertension risk of the user by using the selected blood pressure model. This can reduce impact of different physical conditions of different users on a detection result. In addition, the first electronic device may update the blood pressure model for detecting the degree of the hypertension risk of the user. The updated blood pressure model is most adapted to a user in a current physical condition. This can not only reduce impact of a change of the physical condition of the user on a detection result, to improve accuracy of detecting the degree of the hypertension risk of the user, but also simplify a calibration operation performed by the user on the electronic device when the user detects the degree of the hypertension risk of the user, to improve convenience of performing hypertension risk detection by the user.

With reference to the second aspect, in some embodiments, the first electronic device may be further configured to: collect a second PPG signal of the first user, and send the second PPG signal to the second electronic device. The second electronic device may be configured to select the q blood pressure models from the p blood pressure models by using the second PPG signal. The q blood pressure models are obtained by training q groups of training samples in the p groups of training samples. Features of PPG signals in the q groups of training samples differ least from a feature of the second PPG signal in the p groups of training samples.

With reference to the second aspect, in some embodiments, the second electronic device may be further configured to: obtain physiological feature information of the first user, and select the q blood pressure models from the p blood pressure models by using the physiological feature information. The physiological feature information may include one or more of the following: an age, a gender, a height, and a weight. The q blood pressure models are obtained by training q groups of training samples in the p groups of training samples, and physiological feature information of a user to which PPG signals of the q groups of training samples belong and the physiological feature information of the first user fall within a same value range.

With reference to the second aspect, in some embodiments, the second electronic device may be further configured to: obtain physiological feature information of the first user, and select w blood pressure models from the p blood pressure models by using the physiological feature information. The physiological feature information may include one or more of the following: an age, a gender, a height, and a weight. The w blood pressure models are obtained by training w groups of training samples in the p groups of training samples. Physiological feature information of a user to which PPG signals of the w groups of training samples belong and the physiological feature information of the first user fall within a same value range, where w is a positive integer less than or equal to p and greater than or equal to q. The first electronic device may be further configured to: collect a second PPG signal of the first user, and send the second PPG signal to the second electronic device. The second electronic device may be further configured to select the q blood pressure models from the w blood pressure models by using the second PPG signal. The q blood pressure models are obtained by training q groups of training samples in the w groups of training samples, and features of PPG signals of the q groups of training samples differ least from a feature of the second PPG signal in the w groups of training samples.

With reference to the second aspect, in some embodiments, a time interval between time for collecting the first PPG signal and time for collecting the second PPG signal does not exceed a time length of a second time period.

With reference to the second aspect, in some embodiments, before collecting the second PPG signal of the first user, the first electronic device may be further configured to receive a first user operation. The first user operation indicates the first electronic device to collect the second PPG signal, and send the second PPG signal and a request for obtaining a blood pressure model to the second electronic device.

With reference to the second aspect, in some embodiments, before collecting the second PPG signal of the first user, the first electronic device may be further configured to receive, from the second electronic device, a request for obtaining a PPG signal, where the request for obtaining the PPG signal is sent after the second electronic device receives a second user operation, and the second user operation indicates the second electronic device to select a blood pressure model.

According to a third aspect, this application provides an electronic device. The electronic device may include a PPG signal collection apparatus, a memory, and a processor. The PPG signal collection apparatus is configured to collect a PPG signal. The memory is configured to store a computer program. The processor is configured to invoke the computer program, so that the electronic device performs any possible implementation method in the first aspect.

According to a fourth aspect, an embodiment of this application provides a chip. The chip is used in an electronic device, and the chip includes one or more processors. The processor is configured to invoke computer instructions, so that the electronic device performs any possible implementation method in the first aspect.

According to a fifth aspect, an embodiment of this application provides a computer program product including instructions. When the computer program product runs on a device, the electronic device is enabled to perform any possible implementation method in the first aspect.

According to a sixth aspect, an embodiment of this application provides a computer storage medium, including computer instructions. When the computer instructions are run on an electronic device, the electronic device is enabled to perform any possible implementation method in the first aspect.

It may be understood that the electronic device provided in the third aspect, the chip provided in the fourth aspect, the computer program product provided in the fifth aspect, and the computer storage medium provided in the sixth aspect are all configured to perform the method provided in embodiments of this application. Therefore, for beneficial effects that can be achieved by them, refer to beneficial effects in the corresponding method. Details are not described herein again.

## BRIEF DESCRIPTION OF DRAWINGS

FIG. 1 is an architectural diagram of a communication system 10 according to an embodiment of this application;
FIG. 2 is a schematic diagram of a structure of an electronic device 100 according to an embodiment of this application;
FIG. 3A to FIG. 3F are schematic diagrams of some user interfaces according to embodiments of this application;

FIG. 4A and FIG. 4B each are a schematic diagram of a waveform of a PPG signal according to an embodiment of this application;

FIG. 5 is a flowchart of a hypertension risk detection method according to an embodiment of this application;

FIG. 6 is an architectural diagram of another communication system 20 according to an embodiment of this application;

FIG. 7 is an architectural diagram of another communication system 30 according to an embodiment of this application; and

FIG. 8 is an architectural diagram of another communication system 40 according to an embodiment of this application.

## DESCRIPTION OF EMBODIMENTS

The technical solutions according to embodiments of this application are clearly and completely described in the following with reference to the accompanying drawings. In descriptions of embodiments of this application, "/" means "or" unless otherwise specified. For example, A/B may represent A or B. In this specification, "and/or" describes only an association relationship for describing associated objects and represents that three relationships may exist. For example, A and/or B may represent the following three cases: Only A exists, both A and B exist, and only B exists. In addition, in the descriptions of embodiments of this application, "a plurality of" means two or more.

The terms "first" and "second" mentioned below are merely intended for a purpose of description, and shall not be understood as an indication or implication of relative importance or implicit indication of a quantity of indicated technical features. Therefore, a feature limited by "first " or "second" may explicitly indicate or implicitly include one or more such features. In the descriptions of embodiments of this application, unless otherwise specified, "a plurality of" means two or more.

This application provides a hypertension risk detection method, to detect a degree of a hypertension risk of a user, and in a detection process, the user may not need to perform a complex calibration operation. An electronic device may store a plurality of blood pressure models. The plurality of blood pressure models may be configured to determine the degree of the hypertension risk of the user based on a PPG signal of the user. The plurality of blood pressure models are obtained through training by using a PPG signal dataset. The PPG signal dataset may include a plurality of PPG signals obtained by a plurality of users through detection in a plurality of time periods and blood pressure corresponding to the plurality of PPG signals. One group of training data in the PPG signal dataset may be used for training to obtain one blood pressure model. The group of training data may include a plurality of PPG signals obtained by one user through detection in a period of time (for example, in one week or one month) and blood pressure corresponding to the plurality of PPG signals. The electronic device may collect the PPG signal of the user, and select, from the plurality of blood pressure models based on the PPG signal, one or more blood pressure models that are adapted to the user. The electronic device may determine the degree of the hypertension risk of the user by using the selected blood pressure model.

It can be learned from medical knowledge that blood pressure may include systolic blood pressure and diastolic blood pressure. If systolic blood pressure of the user is higher than 140 millimeters of mercury (mmHg) and/or diastolic blood pressure is higher than 90 mmHg, the user may be diagnosed with hypertension. The degree of the hypertension risk may indicate a probability that the systolic blood pressure of the user is higher than 140 mmHg. A higher probability that the systolic blood pressure of the user is higher than 140 mmHg indicates a higher (that is, a value of the hypertension risk is closer to 1) hypertension risk. A lower probability that the systolic blood pressure of the user is higher than 140 mmHg indicates a lower (that is, a value of the hypertension risk is closer to 0) hypertension risk.

Optionally, the degree of the hypertension risk may alternatively indicate a probability that the diastolic blood pressure of the user is higher than 90 mmHg.

The plurality of blood pressure models may be applicable to most users. In other words, for most users, the electronic device may select, from the plurality of blood pressure models, one or more blood pressure models that are adapted to the user, to determine a degree of a hypertension risk of the user. In this way, the electronic device may select blood pressure models that are adapted to different users, to reduce impact of different physical conditions of different users on a detection result.

Because the physical condition of the user may change, the electronic device may periodically or irregularly update the blood pressure model for detecting the degree of the hypertension risk of the user. The electronic device may select one or more blood pressure models from the plurality of blood pressure models based on a currently collected PPG signal of the user. The one or more blood pressure models are most adapted to a user in a current physical condition. In this way, impact of a change of the physical condition of the user on a detection result can be reduced, thereby improving accuracy of detecting the degree of the hypertension risk of the user. In addition, according to the foregoing hypertension risk detection method, a calibration operation performed by the user on the electronic device when the user detects the degree of the hypertension risk of the user can be simplified, thereby improving convenience of performing hypertension risk detection by the user.

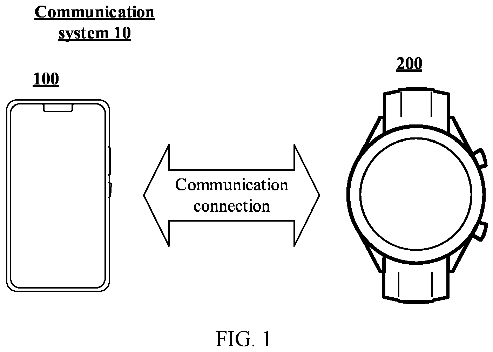

**FIG. 1 is an architectural diagram of an example of a communication system 10 according to this application.**

As shown in FIG. 1, the communication system 10 may include an electronic device 100 and an electronic device 200. The electronic device 100 may be a device, for example, a mobile phone, a tablet computer, a desktop computer, a laptop computer, a handheld computer, a notebook computer, an ultra-mobile personal computer (ultra-mobile personal computer, UMPC), a netbook, or a personal digital assistant (personal digital assistant, PDA). The electronic device 200 may be a wearable device, for example, a watch or a band. Specific types of the electronic device 100 and the electronic device 200 are not limited in this embodiment of this application.

A communication connection is established between the electronic device 100 and the electronic device 200. The communication connection may be a Bluetooth communication connection, a wireless local area network (wireless local area network, WLAN) communication connection, or the like.

In some embodiments, the plurality of blood pressure models mentioned in the foregoing hypertension risk detection method may be stored in the electronic device 100. The plurality of blood pressure models may be obtained by the electronic device 100 through training by using the foregoing PPG signal dataset. The electronic device 200 may be configured to: collect a PPG signal of a user, and send the PPG signal to the electronic device 100. The electronic device 100 may select, from the plurality of blood pressure models based on the PPG signal, one or more blood pressure models that are adapted to the user, and send the blood pressure model to the electronic device 200. The electronic device 200 may detect a degree of a hypertension risk of the user based on the received blood pressure model.

The electronic device 200 may display a detection result (that is, the degree of the hypertension risk) obtained by using the blood pressure model. The electronic device 200 may send the detection result to the electronic device 100. The electronic device 100 may also display the detection result. In other words, the user may view the degree of the hypertension risk of the user by using the electronic device 100 and the electronic device 200.

There may be a binding relationship between the electronic device 100 and the electronic device 200. The electronic device 100 and the electronic device 200 may be bound by logging in to a same account (for example, a Huawei account). Alternatively, the electronic device 100 and the electronic device 200 may be bound in a Bluetooth pairing manner. A method for establishing the binding relationship between the electronic device 100 and the electronic device 200 is not limited in this embodiment of this application.

In some other embodiments, the electronic device 100 may obtain, through training by using the foregoing PPG signal dataset, the plurality of blood pressure models mentioned in the foregoing hypertension risk detection method. The electronic device 100 may store the plurality of blood pressure models. The electronic device 200 may be configured to: collect a PPG signal of a user, and send the PPG signal to the electronic device 100. The electronic device 100 may select, from the plurality of blood pressure models based on the PPG signal, one or more blood pressure models that are adapted to the user. Further, the electronic device 100 may detect a degree of a hypertension risk of the user by using the selected blood pressure model. The electronic device 100 may display a detection result (that is, the degree of the hypertension risk) obtained by using the blood pressure model, and send the detection result to the electronic device 200. The electronic device 200 may display the detection result.

A specific implementation method for establishing the blood pressure model and selecting the blood pressure model that is adapted to the user is described in a subsequent embodiment, and is not described herein.

In the communication system 10 shown in FIG. 1, the electronic device 100 and the electronic device 200 may work together to complete detection of the degree of the hypertension risk of the user. The electronic device 100 may establish the plurality of blood pressure models, and select, from the plurality of blood pressure models, the one or more blood pressure models that are adapted to the user. This can reduce an operation of the electronic device 200, reduce a requirement for a processing capability of the electronic device 200, and facilitate lightweight of the electronic device 200.

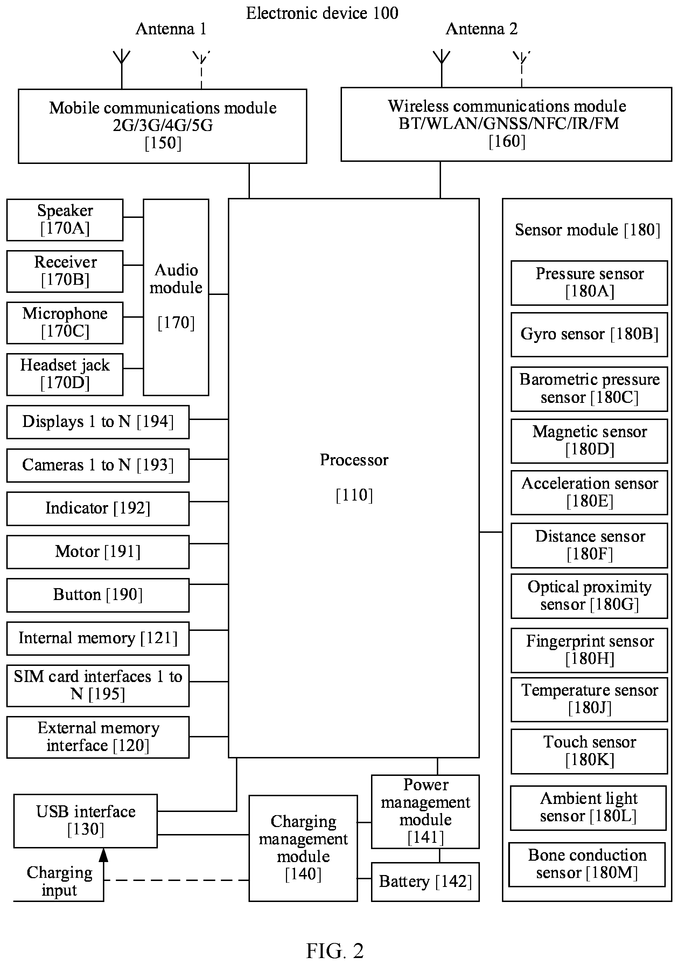

**FIG. 2 is a schematic diagram of a structure of an example of an electronic device 100 according to this application.**

As shown in FIG. 2, the electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communications module 150, a wireless communications module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present disclosure does not constitute a specific limitation on the electronic device 100. In some other embodiments of this application, the electronic device 100 may include more or fewer components than those shown in the figure, combine some components, split some components, or have different component arrangements. The components shown in the figure may be implemented by using hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent devices, or may be integrated into one or more processors.

The controller may be a nerve center and a command center of the electronic device 100. The controller may generate an operation control signal based on instruction operation code and a time sequence signal, to complete control of instruction fetching and instruction execution.

A memory may be further disposed in the processor 110, and is configured to store instructions and data. In some embodiments, the memory in the processor 110 is a cache. The memory may store instructions or data that has been used or is cyclically used by the processor 110. If the processor 110 needs to use the instructions or the data again, the processor may directly invoke the instructions or the data from the memory. This avoids repeated access, reduces waiting time of the processor 110, and improves system efficiency.

The USB interface 130 is an interface that conforms to a USB standard specification, and may be specifically a mini USB interface, a micro USB interface, a USB Type C interface, or the like. The USB interface 130 may be configured to connect to a charger to charge the electronic device 100, may be configured to transmit data between the electronic device 100 and a peripheral device, or may be configured to connect to a headset, to play audio by using the headset. The interface may be further configured to connect to another electronic device, for example, an AR device.

The charging management module 140 is configured to receive a charging input from a charger. The charging management module 140 may further supply power to the electronic device 100 by using the power management module 141 while charging the battery 142.

The power management module 141 is configured to connect to the battery 142, the charging management module 140, and the processor 110. The power management module 141 receives an input of the battery 142 and/or the charging management module 140, and supplies power to the processor 110, the internal memory 121, an external memory, the display 194, the camera 193, the wireless communications module 160, and the like. In some other embodiments, the power management module 141 may alternatively be disposed in the processor 110. In some other embodiments, the power management module 141 and the charging management module 140 may alternatively be disposed in a same device.

A wireless communications function of the electronic device 100 may be implemented by using the antenna 1, the antenna 2, the mobile communications module 150, the wireless communications module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna in a wireless local area network. In some other embodiments, the antenna may be used in combination with a tuning switch.

The mobile communications module 150 may provide a wireless communication solution that is applied to the electronic device 100 and that includes 2G/3G/4G/5G or the like. The mobile communications module 150 may include at least one filter, a switch, a power amplifier, a low noise amplifier (low noise amplifier, LNA), and the like. The mobile communications module 150 may receive an electromagnetic wave through the antenna 1, perform processing such as filtering or amplification on the received electromagnetic wave, and transmit the electromagnetic wave to the modem processor for demodulation. The mobile communications module 150 may further amplify a signal modulated by the modem processor, and convert the signal into an electromagnetic wave for radiation through the antenna 1. In some embodiments, at least some functional modules of the mobile communications module 150 may be disposed in the processor 110. In some embodiments, at least some functional modules of the mobile communications module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 170A, the receiver 170B, or the like), or displays an image or a video through the display 194. In some embodiments, the modem processor may be an independent device. In some other embodiments, the modem processor may be independent of the processor 110, and is disposed in a same device as the mobile communications module 150 or another functional module.

The wireless communications module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communications module 160 may be one or more devices integrating at least one communications processor module. The wireless communications module 160 receives an electromagnetic wave through the antenna 2, performs frequency modulation and filtering processing on an electromagnetic wave signal, and sends a processed signal to the processor 110. The wireless communications module 160 may further receive a to-be-sent signal from the processor 110, perform frequency modulation and amplification on the signal, and convert the signal into an electromagnetic wave for radiation through the antenna 2.

The electronic device 100 may implement a display function through the GPU, the display 194, the application processor, and the like. The GPU is a microprocessor for image processing, and is connected to the display 194 and the application processor. The GPU is configured to perform mathematical and geometric calculation, and render an image. The processor 110 may include one or more GPUs that execute program instructions to generate or change display information.

The display 194 is configured to display an image, a video, or the like. The display 194 includes a display panel. In some embodiments, the electronic device 100 may include one or N displays 194, where N is a positive integer greater than 1.

The electronic device 100 can implement a photographing function by using the ISP, the camera 193, the video codec, the GPU, the display 194, the application processor, and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of a photographing scenario. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP for converting the electrical signal into a digital image signal. The ISP outputs the digital image signal to the DSP for processing. The DSP converts the digital image signal into an image signal in a standard format, for example, RGB or YUV. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 selects a frequency, the digital signal processor is configured to perform Fourier transform or the like on frequency energy.

The video codec is configured to compress or decompress a digital video. The electronic device 100 may support one or more types of video codecs. Therefore, the electronic device 100 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor. The NPU quickly processes input information by referring to a structure of a biological neural network, for example, by referring to a transfer mode between human brain neurons, and may further continuously perform self-learning. Applications such as intelligent cognition of the electronic device 100, for example, image recognition, facial recognition, speech recognition, and text understanding, may be implemented through the NPU.

The external memory interface 120 may be configured to connect to an external storage card, for example, a micro SD card, to extend a storage capability of the electronic device 100. The external storage card communicates with the processor 110 through the external memory interface 120, to implement a data storage function. For example, files such as music and videos are stored in the external storage card.

The internal memory 121 may be configured to store computer-executable program code. The executable program code includes instructions. The processor 110 runs the instructions stored in the internal memory 121, to perform various function applications of the electronic device 100 and data processing. The internal memory 121 may include a program storage area and a data storage area. The program storage area may store an operating system, an application required by at least one function (for example, a sound playing function or an image playing function), and the like. The data storage area may store data (such as audio data and a phone book) and the like that are created during use of the electronic device 100. In addition, the internal memory 121 may include a high-speed random access memory, and may further include a nonvolatile memory, for example, at least one magnetic disk storage device, a flash memory device, or a universal flash storage (universal flash storage, UFS).

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be further configured to code and decode an audio signal. In some embodiments, the audio module 170 may be disposed in the processor 110, or some functional modules of the audio module 170 are disposed in the processor 110.

The speaker 170A, also referred to as a "loudspeaker", is configured to convert an audio electrical signal into a sound signal.

The receiver 170B, also referred to as an "earpiece", is configured to convert an audio electrical signal into a sound signal.

The microphone 170C, also referred to as a "mike" or a "mic", is configured to convert a sound signal into an electrical signal.

The headset jack 170D is configured to connect to a wired headset.

The pressure sensor 180A is configured to sense a pressure signal, and can convert the pressure signal into an electrical signal. In some embodiments, the pressure sensor 180A may be disposed on the display 194.

The gyro sensor 180B may be configured to determine a motion posture of the electronic device 100. In some embodiments, an angular velocity of the electronic device 100 around three axes (that is, axes x, y, and z) may be determined by using the gyro sensor 180B.

The barometric pressure sensor 180C is configured to measure barometric pressure.

The magnetic sensor 180D includes a Hall sensor.

The acceleration sensor 180E may detect accelerations in various directions (usually on three axes) of the electronic device 100. When the electronic device 100 is still, a magnitude and a direction of gravity may be detected. The acceleration sensor 180E may be further configured to identify a posture of the electronic device 100, and is used in applications such as switching between a landscape mode and a portrait mode and a pedometer.

The distance sensor 180F is configured to measure a distance. The electronic device 100 may measure a distance in an infrared manner or a laser manner. In some embodiments, in a photographing scenario, the electronic device 100 may measure a distance by using the distance sensor 180F, to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light by using the light-emitting diode. The electronic device 100 detects infrared reflected light from a nearby object by using the photodiode. When sufficient reflected light is detected, the electronic device 100 may determine that there is an object near the electronic device 100. When insufficient reflected light is detected, the electronic device 100 may determine that there is no object near the electronic device 100.

The ambient light sensor 180L is configured to sense ambient light brightness.

The fingerprint sensor 180H is configured to collect a fingerprint.

The temperature sensor 180J is configured to detect a temperature.

The touch sensor 180K is also referred to as a "touch panel". The touch sensor 180K may be disposed on the display 194, and the touch sensor 180K and the display 194 constitute a touchscreen. The touch sensor 180K is configured to detect a touch operation performed on or near the touch sensor. The touch sensor may transfer the detected touch operation to the application processor to determine a type of a touch event. A visual output related to the touch operation may be provided through the display 194. In some other embodiments, the touch sensor 180K may alternatively be disposed on a surface of the electronic device 100 at a location different from a location of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a human pulse, and receive a blood pressure beating signal. In some embodiments, the bone conduction sensor 180M may alternatively be disposed in the headset, to constitute a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a key input, and generate a key signal input related to user settings and function control of the electronic device 100.

The motor 191 may generate a vibration prompt.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The electronic device 100 interacts with a network through the SIM card, to implement functions such as calling and data communication. In some embodiments, the electronic device 100 uses an eSIM, that is, an embedded SIM card. The eSIM card may be embedded into the electronic device 100, and cannot be separated from the electronic device 100.

For a schematic diagram of a structure of an electronic device 200, refer to the schematic diagram of the structure of the electronic device 100 shown in FIG. 2. Not limited to components in the structure shown in FIG. 2, the electronic device 200 may include more or fewer components.

It should be noted that the electronic device 200 may include an optical emitter and an optical receiver. The electronic device 200 may be worn at a position, for example, a wrist, an abdomen, or a leg of a user, so that the optical emitter may be close to skin of the user. The optical emitter may emit an optical signal to a skin surface. The emitted optical signal may be absorbed, reflected, and scattered by tissues such as blood, skin, muscle, and a bone in a blood vessel. The optical receiver may receive a reflected or scattered optical signal. A volume of the blood in the blood vessel changes during relaxation and contraction of a heart, and an optical signal absorbed by the blood in the blood vessel also changes. Intensity of the optical signal received by the optical receiver also changes with the contraction and relaxation of the heart. The electronic device 200 may generate a PPG signal based on the optical signal received by the optical receiver.

Preferably, light emitted by the optical emitter may be light with a wavelength in a range of 490 nm to 580 nm. A wavelength of the emitted light is not specifically limited in this embodiment of this application.

For a method for generating the PPG signal by the electronic device 200, refer to another method in a conventional technology. This is not limited in this embodiment of this application.

**The following describes schematic diagrams of some user interfaces related to a hypertension risk detection method provided in this application.**

FIG. 3A shows an example of a user interface 210 that is in an electronic device 200 and that is used to trigger the electronic device 200 to perform hypertension risk detection. The user interface 210 may include a title bar 211, a measurement start control 212, and a model update control 213.

The title bar 211 may indicate display content of the user interface 210. The title bar 211 may include a text prompt "hypertension risk screening", to prompt a user that a related control in the user interface 210 may be configured to trigger the electronic device 200 to perform hypertension risk detection.

The measurement start control 212 may be configured to trigger the electronic device 200 to perform hypertension risk detection. In response to a user operation performed on the measurement start control 212, for example, a touch operation, the electronic device 200 may collect a PPG signal of the user. In some embodiments, the user does not use the electronic device 200 for the first time to perform hypertension risk detection, and the electronic device 200 has stored a blood pressure model. Based on the collected PPG signal, the electronic device 200 may determine a degree of a hypertension risk of the user by using the stored blood pressure model.

In some embodiments, the user uses the electronic device 200 for the first time to perform hypertension risk detection. The electronic device 200 has not yet stored a blood pressure model. In response to the user operation performed on the measurement start control 212, the electronic device 200 may collect the PPG signal of the user, and send the PPG signal to an electronic device 100. The electronic device 100 stores a plurality of blood pressure models. The electronic device 100 may select, from the plurality of blood pressure models by using the received PPG signal, one or more blood pressure models that are adapted to the user, and send the blood pressure model to the electronic device 200. The electronic device 200 may store the received blood pressure model, and perform detection by using the blood pressure model. An output of the blood pressure model may be a degree of a hypertension risk of the user.

The PPG signal used when hypertension risk detection is performed may be the PPG signal (namely, the PPG signal used by the electronic device 100 to select the blood pressure model) sent by the electronic device 200 to the electronic device 100. Optionally, the PPG signal used when hypertension risk detection is performed may alternatively be a PPG signal collected after the electronic device 200 receives the blood pressure model.

In other words, when the electronic device 200 detects the degree of the hypertension risk for the first time, the electronic device 100 may select, based on the PPG signal of the user, the blood pressure model that is adapted to the user. The electronic device 200 performs detection by using the blood pressure model, so that accuracy of a detection result can be improved.

In some embodiments, the user does not use the electronic device 200 for the first time to perform hypertension risk detection. The electronic device 200 has stored a blood pressure model. The electronic device 200 may determine a degree of a hypertension risk of the user by using the collected PPG signal and the stored blood pressure model.

In some embodiments, the electronic device 200 may automatically detect a degree of a hypertension risk of the user at an interval of a preset time period (for example, 30 minutes). The electronic device 200 may store both a detection result obtained by manually triggering, by the user, the electronic device 200 to perform hypertension risk detection and a detection result obtained by automatically performing hypertension risk detection by the electronic device 200. A length of the preset time period is not limited in this embodiment of this application.

The model update control 213 may be configured to trigger the electronic device 200 to update the blood pressure model stored in the electronic device 200. In response to a user operation performed on the model update control 213, for example, a touch operation, the electronic device 200 may collect the PPG signal of the user, and send the PPG signal to the electronic device 100. The electronic device 100 may select, from the plurality of blood pressure models by using the received PPG signal, one or more blood pressure models that are adapted to the user, and send the selected blood pressure model to the electronic device 200. The electronic device 200 may replace the stored blood pressure model with the received blood pressure model.

In other words, the user may manually update the blood pressure model that is stored in the electronic device 200 and that is for hypertension risk detection. The updated blood pressure model is more adapted to a current physical condition of the user, and can more accurately detect the degree of the hypertension risk of the user. In the foregoing method for updating the blood pressure model, the user only needs to perform the user operation (for example, the touch operation) on the model update control 213. Compared with calibrating a hypertension risk detection device by using a professional hypertension risk detection device or by going to hospital for detection to obtain blood pressure with high accuracy, the foregoing method for updating the blood pressure model is simpler, and can not only reduce impact of a change of a physical condition of the user on the detection result, but also improve convenience of performing hypertension risk detection by the user.

In some embodiments, alternatively, the electronic device 200 may periodically or irregularly update the blood pressure model stored in the electronic device 200. When the electronic device 200 performs hypertension risk detection at different time, the blood pressure model used by the electronic device 200 may be most adapted to the user in the current physical condition. This can reduce the impact of the change of the physical condition of the user on the detection result, to improve the accuracy of the detection result. For a method for updating, by the electronic device 200, the blood pressure model stored in the electronic device 200, refer to the foregoing embodiment.

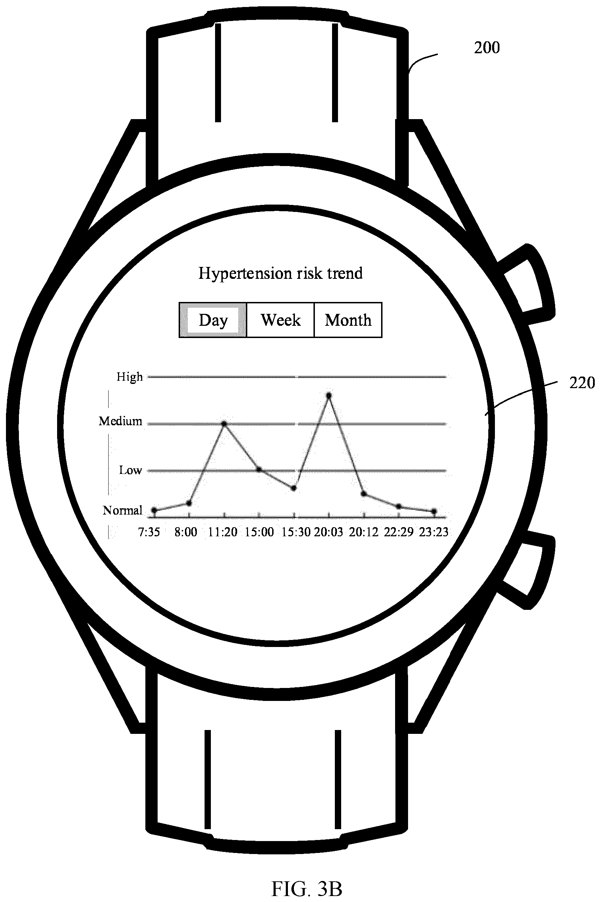

FIG. 3B shows an example of a user interface 220 in which the electronic device 200 displays a detection result.

The user interface 220 may be used to display the detection result obtained by the electronic device 200 by performing hypertension risk detection. For example, the electronic device 200 may draw, as a line chart shown in FIG. 3B, a degree of a hypertension risk obtained through detection in time T (for example, one day, one week, or one month). For example, a hypertension risk trend of the user and a time period in which blood pressure of the user is high may be seen from a plurality of detection results in one day. The user may adjust mood, a life schedule, and the like of the user with reference to the detection result, to reduce a risk that may be caused by hypertension to the user.

Not limited to the line chart shown in FIG. 3B, the electronic device 200 may alternatively present the detection result in another representation form.

The user interface 210 and the user interface 220 are merely examples for description of embodiments of this application, and shall not constitute a limitation on this application.

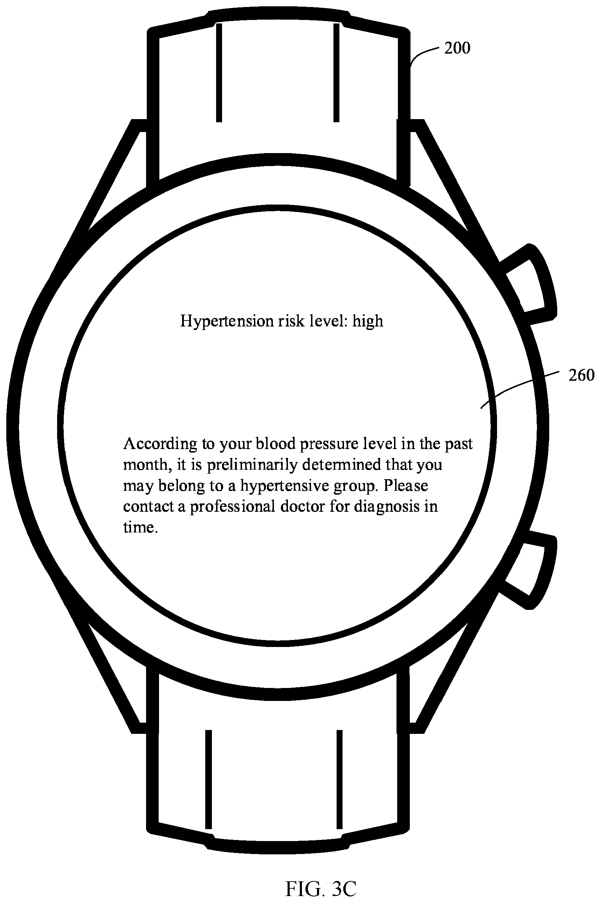

FIG. 3C shows an example of a user interface 260 in which the electronic device 200 displays a detection result.

As shown in FIG. 3C, the electronic device 200 may provide a hypertension risk level of the user in the time T by comprehensively considering detection results of the user in the time T (for example, one day, one week, or one month). For example, 85% of detection results of the electronic device 200 in one month indicate that a value of the hypertension risk of the user is greater than 0.7. The electronic device 200 may provide that a hypertension risk level of the user in the month is high. In addition, the electronic device 200 may display a related suggestion on the user interface 260 based on the hypertension risk level. For example, the prompt suggestion may be: "According to your blood pressure level in the past month, it is preliminarily determined that you may belong to a hypertensive group. Please contact a professional doctor for diagnosis in time". Specific content of the prompt suggestion is not limited in this embodiment of this application.

FIG. 3D shows an example of a user interface 230 that is in an electronic device 100 and that is used to trigger an electronic device 200 to perform hypertension risk detection. The user interface 230 may include a user information area 231, a measurement start control 232, and a model update control 233.

The user information area 231 may include information about a user, for example, a nickname, an age, a gender, a height, and a weight. The user information area 231 may include more or less information. This is not limited in this embodiment of this application.

The measurement start control 232 may be configured to trigger the electronic device 100 to indicate the electronic device 200 to perform hypertension risk detection. In response to a user operation performed on the measurement start control 232, for example, a touch operation, the electronic device 100 may send an instruction to the electronic device 200. The instruction may instruct the electronic device 200 to perform hypertension risk detection. When receiving the instruction, the electronic device 200 may collect a PPG signal of the user, and determine a degree of a hypertension risk of the user by using a stored blood pressure model. The electronic device 200 may send a detection result to the electronic device 100. The electronic device 100 may store the received detection result.

In some embodiments, the user uses the electronic device 100 for the first time to indicate the electronic device 200 to perform hypertension risk detection. The electronic device 200 has not yet stored the blood pressure model. When receiving the instruction that instructs the electronic device 200 to perform hypertension risk detection, the electronic device 200 may collect the PPG signal of the user, send the PPG signal to the electronic device 100, and request a blood pressure model. When receiving a message for requesting the blood pressure model, the electronic device 100 may select, from a plurality of blood pressure models by using the received PPG signal, one or more blood pressure models that are adapted to the user, and send the blood pressure model to the electronic device 200.

The model update control 233 may be configured to trigger the electronic device 100 to update the blood pressure model stored in the electronic device 200. In response to a user operation performed on the model update control 233, for example, a touch operation, the electronic device 100 may send an instruction to the electronic device 200. The instruction may instruct the electronic device 200 to collect the PPG signal of the user. When receiving the instruction, the electronic device 200 may collect the PPG signal of the user, and send the PPG signal to the electronic device 100. The electronic device 100 may select, from a plurality of blood pressure models based on the received PPG signal, one or more blood pressure models that are adapted to the user, send the blood pressure model to the electronic device 200, and indicate the electronic device 200 to update the model. The electronic device 200 may replace the stored blood pressure model with the received blood pressure model.

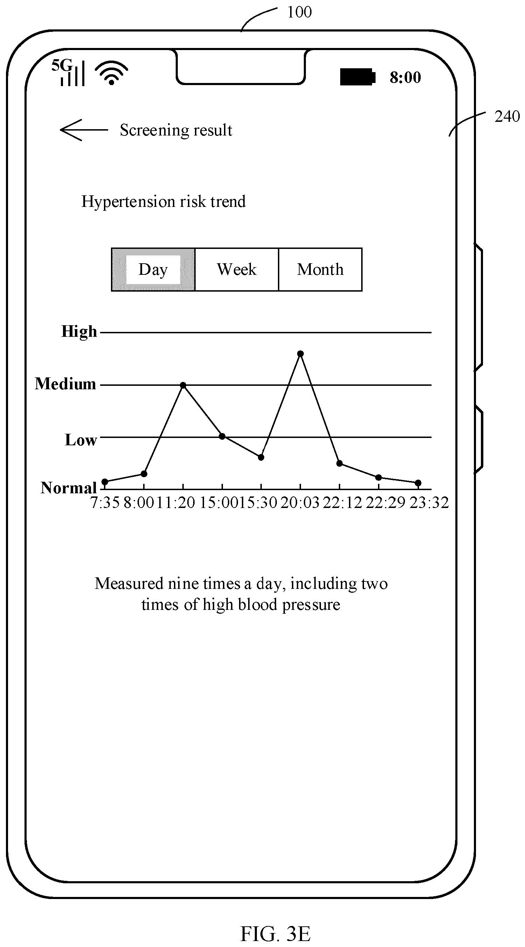

FIG. 3E shows an example of a user interface 240 in which the electronic device 100 displays a detection result.

After performing detection by using the blood pressure model, the electronic device 200 may send the obtained detection result to the electronic device 100. The electronic device 100 may draw, as a line chart shown in FIG. 3B, a degree of a hypertension risk obtained through detection in time T (for example, one day, one week, or one month). A representation manner of the detection result in the user interface 240 is not limited in this embodiment of this application.

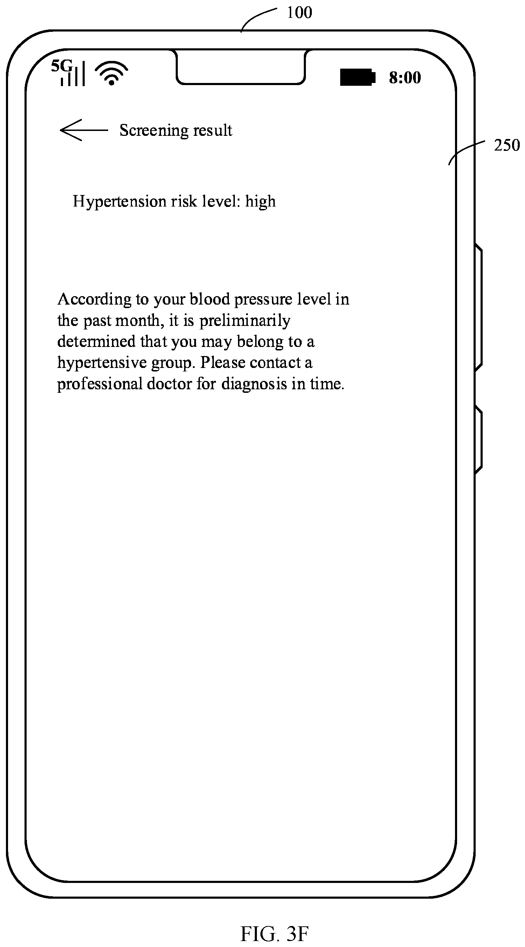

FIG. 3F shows an example of a user interface 250 in which the electronic device 100 displays a detection result.

As shown in FIG. 3F, the electronic device 100 may provide a hypertension risk level of the user in the time T by comprehensively considering detection results of the user in the time T (for example, one day, one week, or one month). For example, 85% of detection results received by the electronic device 100 in one month indicate that a value of the hypertension risk of the user is greater than 0.7. The electronic device 100 may provide that a hypertension risk level of the user in the month is high. In addition, the electronic device 100 may display a related suggestion on the user interface 250 based on the hypertension risk level. For example, the prompt suggestion may be: "According to your blood pressure level in the past month, it is preliminarily determined that you may belong to a hypertensive group. Please contact a professional doctor for diagnosis in time". Specific content of the prompt suggestion is not limited in this embodiment of this application.

The user interface 230, the user interface 240, the user interface 250, and the user interface 260 are merely examples for description of embodiments of this application, and shall not constitute a limitation on this application.

In some embodiments, when selecting, from the plurality of blood pressure models, the one or more blood pressure models that are adapted to the user, the electronic device 100 may alternatively use the information (for example, the gender, the age, the height, and the weight) about the user in addition to the PPG signal.

Specifically, the plurality of blood pressure models may include a model attribute. A model attribute of one blood pressure model may be determined based on information about a user in one group of training data that is trained to obtain the blood pressure model and that is in a PPG signal dataset. For example, the information about the user in the group of training data that is trained to obtain the blood pressure model includes: a gender of a female, an age of 20, a height of 160 cm, and a weight of 50 kg. The model attribute of the blood pressure model includes: a gender of a female, an age class in a range of 20 to 30 years old, a height class in a range of 150 to 170 cm, and a weight class in a range of 40 to 60 kg.

Range division of the age class, the height class, and the weight class is not limited in this embodiment of this application.

When selecting the blood pressure model, the electronic device 100 may obtain the information about the user. Based on the information about the user, the electronic device 100 may select, from the plurality of blood pressure models, several blood pressure models that match the information about the user. If the information about the user is included in a value range of each type of information in a model attribute of a blood pressure model, the model attribute of the blood pressure model matches the information about the user. The electronic device 100 may compare the information about the user with the model attributes of the plurality of blood pressure models, to determine the several blood pressure models.

In some embodiments, based on the blood pressure model selected by using the information about the user, the electronic device 100 further selects, from the several blood pressure models by using the PPG signal, one or more blood pressure models that are adapted to the user.

In a possible implementation, before the electronic device 200 performs hypertension risk detection for the first time, the electronic device 100 may display a user information entry interface to obtain the information about the user, for example, the gender, the age, the height, and the weight. A method for obtaining the information about the user by the electronic device 100 is not limited in this embodiment of this application.

Different blood pressure models are adapted to people of different genders, different ages, different heights, and different weights. Usually, a higher age indicates a higher value of a hypertension risk. Blood pressure models that are adapted to users of a same age class may be similar. The electronic device 100 adds the information about the user to select the blood pressure model that is adapted to the user, so that efficiency of selecting the blood pressure model that is adapted to the user can be improved.

**The following specifically describes a method for establishing a blood pressure model provided in an embodiment of this application.**

1. Data preprocessing

A model establishment device may establish a model library by using a PPG signal dataset. The model library may include a plurality of blood pressure models. One of the plurality of blood pressure models may be obtained by using one group of training data in the PPG signal dataset. The group of training data may include a plurality of PPG signals obtained by one user through detection in a period of time (for example, in one week or one month) and blood pressure corresponding to the plurality of PPG signals.

In the period of time, a physical condition of a person usually does not change significantly. The plurality of PPG signals obtained through detection in the period of time and the blood pressure corresponding to the plurality of PPG signals may reflect the physical condition of the person in the period of time. The plurality of PPG signals obtained through detection in the period of time and the blood pressure corresponding to the plurality of PPG signals may be for establishing one blood pressure model. In this case, the blood pressure model may be adapted to a user whose physical condition is a first physical condition. The first physical condition is a physical condition reflected by the plurality of PPG signals obtained through detection in the period of time and the blood pressure corresponding to the plurality of PPG signals. In other words, when the physical condition of the user is the first condition, the electronic device 200 may accurately detect a degree of a hypertension risk of the user by using the blood pressure model.

The model establishment device may preprocess the group of training data. Specifically, the model establishment device may filter a plurality of PPG signals in one group of training data, and label the plurality of PPG signals based on blood pressure corresponding to the plurality of PPG signals.

The model establishment device filters the PPG signal, so that a noise part in the PPG signal can be filtered out, to improve accuracy of blood pressure model detection. The foregoing filtering method is not limited in this embodiment of this application. For a specific implementation method for filtering the PPG signal by the model establishment device, refer to an implementation method in a conventional technology. Details are not described in this embodiment of this application.

The blood pressure model may be configured to detect the degree of the hypertension risk of the user. The degree of the hypertension risk may indicate a probability that systolic blood pressure of the user is higher than 140 mmHg, or may indicate a probability that diastolic blood pressure of the user is higher than 90 mmHg. In this embodiment of this application, an example in which the degree of the hypertension risk may indicate the probability that the systolic blood pressure of the user is higher than 140 mmHg is used for description. The model establishment device may determine a label value corresponding to each PPG signal by comparing systolic blood pressure corresponding to the plurality of PPG signals with 140 mmHg. If systolic blood pressure corresponding to one PPG signal is higher than 140 mmHg, a label value corresponding to the PPG signal is 1. If systolic blood pressure corresponding to one PPG signal is lower than or equal to 140 mmHg, a label value corresponding to the PPG signal is 0.

2. PPG signal feature extraction

A feature of the PPG signal is closely related to a cardiovascular status of the user. The model establishment device may perform feature extraction on the PPG signal, and establish a blood pressure model based on an extracted feature.

In some embodiments, the feature obtained by performing feature extraction on the PPG signal by the model establishment device may include a mean (mean) of rate-to-rate intervals (rate-to-rate intervals, RRIs), a median (median) of the RRIs, a minimum (minimum, min) value of the RRIs, a coefficient of variation of rate-to-rate intervals (coefficient of variation of RR intervals, CVRR), a standard deviation of normal-to-normal intervals (standard deviation of normal-to-normal intervals, SDNN), a main peak value of the PPG signal, a rise time interval of the PPG signal, a fall time interval of the PPG signal, and the like.

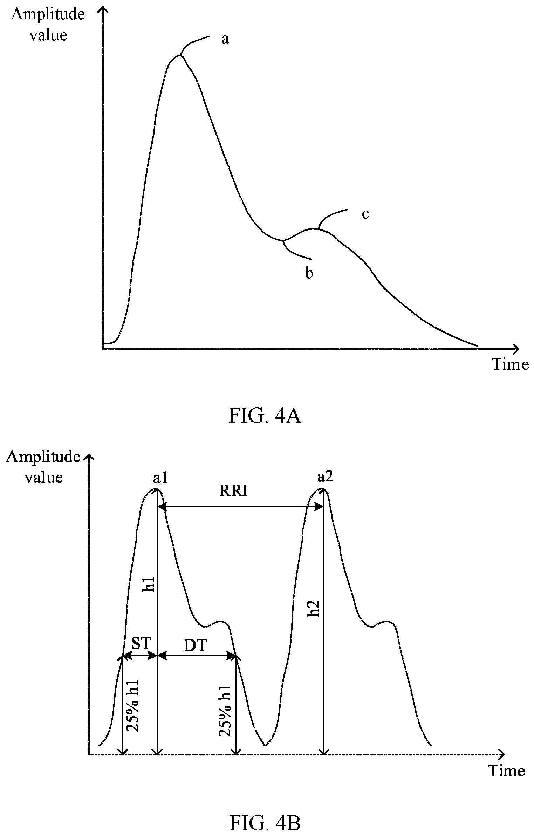

FIG. 4A shows an example of a waveform of one beat signal in one PPG signal.

A heart contracts and relaxes rhythmically, and blood is ejected from the heart into a blood vessel and flows from the blood vessel back to the heart. The blood vessel is an elastic cavity, and the blood flows in the blood vessel to form a pulse wave. A waveform of the PPG signal may include a rising segment and a falling segment. Based on a process of cardiac ejection and blood propagation in the blood vessel, one beat signal in the PPG signal may include three feature points shown in FIG. 4A: a main peak point (marked as a), a dicrotic wave trough point (marked as b), and a dicrotic wave peak point (marked as c). In the beat signal shown in FIG. 4A, a rising segment of the beat signal is before the point c. A falling segment of the beat signal is after the point c. Before the point c, an aorta opens, and pressure in the aorta rises to the point c as the heart ejects the blood into the aorta. The point c is a peak of the beat signal. The blood then erupts from a left ventricle, a right ventricle begins to fill, an atrioventricular valve opens, and the blood bounces back to form a point f. After a point d, the aortic blood bounces back as the heart relaxes, the arterial pressure rises slightly, and the blood vessel dilates again to form a dicrotic wave.

One PPG signal may include a plurality of beat signals. A time interval of one beat signal is time consumed by the heart to beat once. In other words, a quantity of beat signals included in one PPG signal may indicate a quantity of times of heart beating in a time period in which the PPG signal is obtained.

The quantity of beat signals included in the PPG signal may be determined based on time for obtaining the PPG signal through collecting. Longer time for obtaining the PPG signal through collecting indicates a larger quantity of beat signals included in the PPG signal. A length of time for obtaining one PPG signal through collecting is not limited in this embodiment of this application.

FIG. 4B shows an example of a partial waveform in one PPG signal.

As shown in FIG. 4B, a time interval between main peak points of two consecutive beat signals is an RRI. The model establishment device may calculate all RRIs in one PPG signal and further obtain a mean, a median, and min of these RRIs.

The CVRR can reflect a change of time consumed by a plurality of times of heart beating. The CVRR may be obtained through calculation by using the following formula (1):

$$CVRR = \frac{\sqrt{\dfrac{\sum_{i=1}^{n}\left(RRI_i - mean\right)^2}{n-1}}}{mean} \quad (1)$$

A value of n is a quantity of RRIs in one PPG signal. $RRI_i$ is a value of an $i^{th}$ RRI. i is an integer ranging from 1 to n.

The SDNN may represent complexity of heart rate variability. Usually, a larger value of the SDNN indicates higher complexity of the heart rate variability, and indicates a healthier human body. A smaller value of the SDNN indicates lower complexity of the heart rate variability, and indicates a poorer health status of the human body. The SDNN may be obtained through calculation by using the following formula (2):

$$SDNN = \sqrt{\dfrac{\sum_{i=1}^{n}\left(RRI_i - mean\right)^2}{n-1}} \quad (2)$$

It can be learned from FIG. 4B that a main peak value of one beat signal is an amplitude value corresponding to the beat signal at a main peak point. For example, a main peak point of a beat signal on a left side in FIG. 4B is a point a1. An amplitude value corresponding to the point a1 is h1. A main peak value of the beat signal on the left side is h1. A main peak value of a PPG signal may be a mean (or a maximum value, a minimum value, or a median) of main peak values of all beat signals in one PPG signal.

A rise time interval (ST) of one beat signal may be a time interval between a first point whose amplitude value is q in a rising segment of the beat signal and a main peak point of the beat signal. A fall time interval (DT) of one beat signal may be a time interval between a main peak point of the beat signal and a last point whose amplitude value is q in a falling segment of the beat signal. A value of q may be 25% of the main peak value of the beat signal. The value of q is not limited in this embodiment of this application. Alternatively, q may be another value less than the main peak value. A rise time interval of the PPG signal may be a mean (or a maximum value, a minimum value, or a median) of ST of all beat signals in one PPG signal. A fall time interval of the PPG signal may be a mean (or a maximum value, a minimum value, or a median) of DT of all beat signals in one PPG signal.

Not limited to the foregoing described feature of the PPG signal, when performing feature extraction on the PPG signal, the model establishment device may further extract more or fewer features to establish the blood pressure model.

A method for performing feature extraction on the PPG signal by the model establishment device may be, for example, a differential method, a curvature method, or a wavelet transform zero-crossing method. The foregoing feature extraction method is not limited in this embodiment of this application. For a specific implementation process in which the model establishment device performs feature extraction on the PPG signal, refer to an implementation process in a conventional technology. Details are not described in this application.

3. Blood pressure model training

The blood pressure model may be, for example, a linear model or an xgboost model. A type of the blood pressure model is not limited in this embodiment of this application.

Herein, the blood pressure model is used as a linear model for specific description. For one blood pressure model established by the model establishment device by using the feature of the PPG signal, refer to the following formula (3):

$$M = \sum\nolimits_{j=1}^{k} e_j x_j + \mu \quad (3)$$

A value of $k$ is a quantity of features of the PPG signal used by the model establishment device when establishing the blood pressure model. $x_j$ is a $j^{th}$ feature of the PPG signal. $e_j$ is a coefficient of the $j^{th}$ feature of the PPG signal. $j$ is an integer ranging from 1 to $k$. $e_1, e_2, \cdots, e_k$, and $\mu$ are constants.

The model establishment device may train the blood pressure model by using data in the PPG signal dataset, and determine $e_1, e_2, \cdots, e_k$, and $\mu$ in the model.

The model establishment device may obtain one blood pressure model through training by using one group of training data in the PPG signal dataset. The group of training data may include a plurality of PPG signals obtained by one user through detection in a period of time (for example, in one week or one month) and blood pressure corresponding to the plurality of PPG signals. The blood pressure corresponding to the plurality of PPG signals is blood pressure of a measured person when the plurality of PPG signals are collected. The blood pressure corresponding to the plurality of PPG signals may be for determining label values corresponding to the plurality of PPG signals.

A process of training a blood pressure model by using one group of training data is inputting feature values of a plurality of PPG signals in the group of training data into the blood pressure model, so that a difference between a value of a hypertension risk output by the blood pressure model and label values corresponding to the plurality of PPG signals is narrowed.

A method for training the blood pressure model by the model establishment device is not limited in this embodiment of this application. For a specific training process of the blood pressure model, refer to a process of training a model, for example, the linear model or the xgboost model in the conventional technology.

The model establishment device may obtain the model library through training by using a plurality of groups of training data in the PPG signal dataset. The model library may include a plurality of blood pressure models. The plurality of blood pressure models may be applicable to most users.

The model establishment device may be the electronic device 100 in embodiments of this application, or may be another electronic device. If the model establishment device is the electronic device 100, the electronic device 100 may store the model library and a feature value of a PPG signal corresponding to each blood pressure model in the model library. A feature value of a PPG signal corresponding to one blood pressure model is a feature value of a PPG signal in one group of training data that is for training the blood pressure model. The feature value of the PPG signal in the group of training data may be a mean, a maximum value, a minimum value, or a median of same features of a plurality of PPG signals in the group of training data, or a value obtained by processing each feature of the plurality of PPG signals by using another operation method. If the model establishment device is an electronic device other than the electronic device 100, the another electronic device may send, to the electronic device 100, the model library and a feature value of a PPG signal corresponding to each blood pressure model in the model library. The electronic device 100 may store the received model library and the received feature value of the PPG signal corresponding to each blood pressure model in the model library.

**The following specifically describes a method for selecting a blood pressure model that is adapted to a user according to an embodiment of this application.**

In some embodiments, an electronic device 100 may select, by using a feature similarity measurement method, the blood pressure model that is adapted to the user.

The electronic device 100 may obtain a PPG signal of the user. The electronic device 100 may filter the PPG signal, to filter out noise in the PPG signal. Further, the electronic device 100 may extract a feature of the PPG signal on which filtering processing is performed. For a feature extraction method, refer to the descriptions of the foregoing embodiments, and details are not described herein again. The electronic device 100 may calculate a feature similarity between a feature value of the PPG signal and a feature value of a PPG signal corresponding to each blood pressure model in a model library, and select, from the model library by using the feature similarity, the blood pressure model that is adapted to the user. Details are as follows.

1. Feature similarity calculation

In a possible implementation, the electronic device 100 may calculate the feature similarity by calculating a maximum mean discrepancy (maximum mean discrepancy, MMD) between the feature value of the PPG signal of the user and the feature value of the PPG signal corresponding to each blood pressure model in the model library. For a calculation method of the MMD, refer to the following formula (4):

$$d\left(X_{U}, X_{M}\right) = \left\| \frac{1}{k} \sum_{j=1}^{k} x_{U,j} - \frac{1}{k} \sum_{j=1}^{k} x_{M,j} \right\|^{2} \quad (4)$$

$X_U$ is a feature value vector of the PPG signal of the user, and $X_U = [x_{U,1}, x_{U,2}, \cdots, x_{U,k}]$. $x_{U,j}$ is the $j$th feature of the PPG signal of the user. $X_M$ is a feature value vector of the PPG signal corresponding to each blood pressure model in the model library, and $X_M = [x_{M,1}, x_{M,2}, \cdots, x_{M,k}]$. $x_{M,j}$ is a $j$th feature of the PPG signal corresponding to each blood pressure model in the model library. A value of $k$ is a quantity of features extracted when feature extraction is performed on the PPG signal. $j$ is an integer ranging from 1 to $k$.

The feature similarity is $d(X_U, X_M)$.

A method for calculating the feature similarity is not limited in this embodiment of this application. For example, the electronic device 100 may alternatively calculate the feature similarity by using a similarity measurement algorithm, for example, calculating a Euclidean distance, a Manhattan distance, or a KL divergence.

2. Model selection

When obtaining the feature similarity between the user and a plurality of blood pressure models in the model library, the electronic device 100 may select one or more blood pressure models whose feature similarity ranks first. A feature of a PPG signal corresponding to the one or more blood pressure models whose feature similarity ranks first has a smallest difference from the feature of the PPG signal of the user in the model library.

In a possible implementation, the electronic device 100 may compare the feature similarity with a similarity threshold. If a quantity of blood pressure models whose feature similarity is greater than the similarity threshold is greater than or equal to a preset quantity, the electronic device 100 may select a blood pressure model whose feature similarity is greater than the similarity threshold. If a quantity of blood pressure models whose feature similarity is greater than the similarity threshold is less than a preset quantity, the electronic device 100 may select a blood pressure model whose feature similarity is the largest. The similarity threshold and the preset quantity may be set based on experience. The similarity threshold and the preset quantity are not limited in this embodiment of this application.

In some embodiments, the electronic device 100 may select, by using a ranking algorithm, the blood pressure model that is adapted to the user.

Specifically, the electronic device 100 may establish a ranking model (for example, a learning to rank ranking model). The ranking model may rank the plurality of blood pressure models in the model library based on the PPG signal of the user. A blood pressure model that ranks first has a high degree of adaptation to the user. A blood pressure model that ranks behind has a low degree of adaptation to the user.

The ranking model may be obtained by training a plurality of groups of training data. One group of training data includes a feature value extracted from one PPG signal, feature values of PPG signals corresponding to the plurality of blood pressure models in the model library, and correct ranking of the plurality of blood pressure models in the model library. The correct ranking of the plurality of blood pressure models in the model library may be determined based on the feature value extracted from the PPG signal and a value of a hypertension risk corresponding to the PPG signal. If systolic blood pressure corresponding to one PPG signal is higher than 140 mmHg, a value of a hypertension risk corresponding to the PPG signal is 1. If systolic blood pressure corresponding to one PPG signal is lower than or equal to 140 mmHg, a value of a hypertension risk corresponding to the PPG signal is 0. A smaller difference between a value of a hypertension risk obtained by inputting the feature value extracted from the PPG signal into one blood pressure model and the value of the hypertension risk corresponding to the PPG signal indicates a higher ranking of the blood pressure model in the correct ranking.

In a training process, the ranking model may learn a similarity between a feature value extracted from one PPG signal in one group of training data and the feature values of the PPG signals corresponding to the plurality of blood pressure models in the model library. The training process of the ranking model is a process of narrowing a difference between the correct ranking and ranking results that are of the plurality of blood pressure models and that are output by the ranking model.

The electronic device 100 may obtain the PPG signal of the user, and input the feature extracted from the PPG signal into the ranking model, to obtain the ranking results of the plurality of blood pressure models in the model library. The electronic device 100 may select one or more blood pressure models that rank first in the ranking result.

Not limited to the foregoing feature similarity measurement method and ranking algorithm, the electronic device 100 may alternatively determine, by using another method, a matching degree between the feature value of the PPG signal of the user and the feature value of the PPG signal corresponding to each blood pressure model, and select one or more blood pressure models with a high matching degree.

In some embodiments, the electronic device 100 may select, in combination with the PPG signal of the user and information about the user, the blood pressure model that is adapted to the user.

The electronic device 100 may store the model library, the feature values of the PPG signals corresponding to the plurality of blood pressure models in the model library, and model attributes of the plurality of blood pressure models in the model library. A model attribute of one blood pressure model may be determined based on information about a user in one group of training data that is trained to obtain the blood pressure model and that is in a PPG signal dataset. The information about the user may include but is not limited to a gender, an age, a height, and a weight.

The electronic device 100 may first select several blood pressure models from the plurality of blood pressure models in the model library by using the information about the user. Further, the electronic device 100 may select, from the several blood pressure models by using the PPG signal of the user, one or more blood pressure models that are adapted to the user. For a specific method for selecting the blood pressure model by the electronic device 100 by using the information about the user and the PPG signal, refer to the foregoing embodiments. Details are not described herein again.

A method for selecting a blood pressure model by using a PPG signal is more complex than a method for selecting a blood pressure model by using information about a user. The electronic device 100 may perform preliminary screening on the plurality of blood pressure models in the model library by using the information about the user, and further perform screening, by using the PPG signal of the user, on the blood pressure models obtained through preliminary screening, so that efficiency of selecting the blood pressure model that is adapted to the user can be improved.

**Based on the foregoing methods for establishing and selecting the blood pressure model, the following specifically describes a hypertension risk detection method provided in an embodiment of this application.**

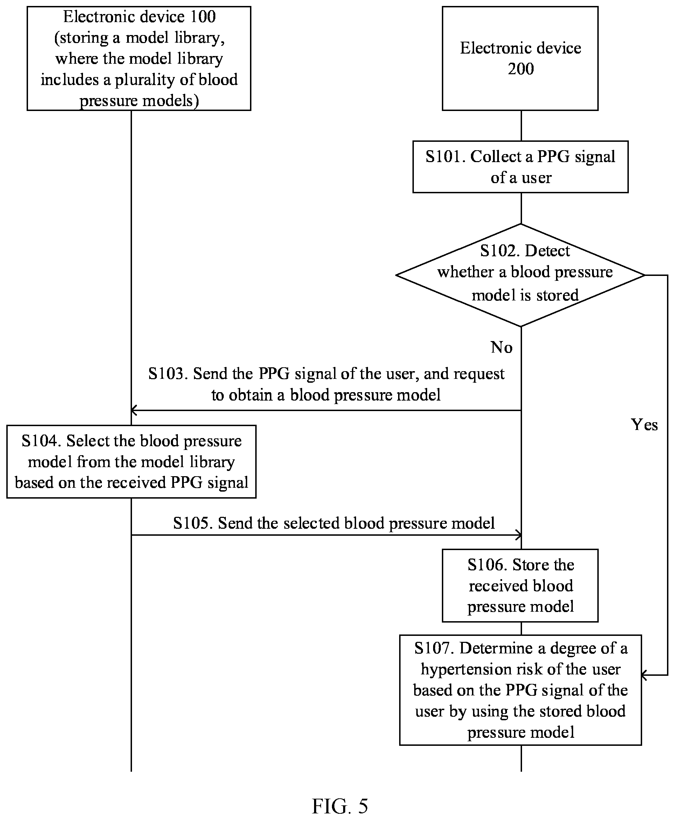

FIG. 5 is a flowchart of an example of a hypertension risk detection method. The method may include steps S101 to S107.

S101. An electronic device 200 may collect a PPG signal of a user.

In some embodiments, the electronic device 200 may receive a user operation for triggering the electronic device 200 to perform hypertension risk detection. The user operation may be, for example, the touch operation performed on the measurement start control 212 shown in FIG. 3A. When receiving the user operation, the electronic device 200 may start to collect the PPG signal of the user.

In some embodiments, the electronic device 200 may automatically perform hypertension risk detection at an interval of a preset time period. In a process of performing hypertension risk detection, the electronic device 200 may collect the PPG signal of the user.

A time length in which the electronic device 200 collects the PPG signal of the user is not limited in this embodiment of this application.

S102. The electronic device 200 may detect whether the electronic device 200 stores a blood pressure model.

When storing no blood pressure model, the electronic device 200 may perform step S103.

When storing a blood pressure model, the electronic device 200 may perform step S107.

In some embodiments, when hypertension risk detection is not performed, the electronic device 200 does not store a blood pressure model. In this case, when performing hypertension risk detection, the electronic device 200 may request to obtain the blood pressure model from an electronic device 100 (that is, perform step S103).

In other words, when the user performs hypertension risk detection by using the electronic device 200 for the first time, the electronic device 200 may request, from the electronic device 100, to obtain a blood pressure model that is adapted to the user.

In some embodiments, when hypertension risk detection is performed at least once, the electronic device 200 stores a blood pressure model. In this case, the electronic device 200 may perform hypertension risk detection (that is, perform step S107) by using the stored blood pressure model.

In some embodiments, before the electronic device 200 performs hypertension risk detection for the first time, the electronic device 200 may request, from an electronic device 100, to obtain a blood pressure model that is adapted to the user, and periodically or irregularly update the blood pressure model stored in the electronic device 200.

In other words, when hypertension risk detection is not performed, the electronic device 200 may store the blood pressure model. In this case, the electronic device 200 may perform hypertension risk detection (that is, perform step S107) by using the stored blood pressure model.

It should be noted that, after the electronic device 200 performs hypertension risk detection for the first time, the electronic device 200 may periodically or irregularly update the blood pressure model stored in the electronic device 200.

S103. If no blood pressure model is stored in the electronic device 200, the electronic device 200 may send the PPG signal of the user to the electronic device 100, to request to obtain a blood pressure model.

In some embodiments, the electronic device 200 may send, to the electronic device 100, the PPG signal collected in step S101.

In some other embodiments, the electronic device 200 may filter the PPG signal collected in step S101, and perform feature extraction on the filtered PPG signal to obtain a feature value of the PPG signal. The electronic device 200 may send the feature value of the PPG signal to the electronic device 100.

S104. The electronic device 100 may select a blood pressure model from a model library based on the received PPG signal.

The electronic device 100 stores the model library. The model library includes a plurality of blood pressure models. The electronic device 100 may further store a feature value of a PPG signal corresponding to each blood pressure model. For a method for establishing the model library, refer to the foregoing embodiments.

The electronic device 100 may select the blood pressure model from the model library based on the received PPG signal. Alternatively, the electronic device 100 may select the blood pressure model from the model library in combination with the received PPG signal and information about the user to which the PPG signal belongs. The information (for example, a gender, an age, a height, and a weight) about the user to which the PPG signal belongs may be sent by the electronic device 200 to the electronic device 100. Optionally, the information about the user to which the PPG signal belongs may be obtained by displaying a user information entry interface by the electronic device 100 and by using a user input received on the user information entry interface. A method for obtaining the information about the user by the electronic device 200 is not limited in this embodiment of this application.

For a method for selecting the blood pressure model from the model library by the electronic device 100, refer to the foregoing embodiments. Details are not described herein again.

S105. The electronic device 100 may send the selected blood pressure model to the electronic device 200.

There may be one or more selected blood pressure models.

S106. The electronic device 200 may store the received blood pressure model.

S107. The electronic device 200 may determine a degree of a hypertension risk of the user based on the PPG signal of the user by using the stored blood pressure model.

The electronic device 100 may filter the PPG signal of the user, and perform feature extraction on the filtered PPG signal to obtain the feature value of the PPG signal.

In some embodiments, the electronic device 200 stores one blood pressure model. The electronic device 200 may input the feature value of the PPG signal into the blood pressure model, to obtain a value of the hypertension risk of the user. A higher value of the hypertension risk indicates a higher probability that systolic blood pressure of the user is higher than 140 mmHg and a higher risk that the user suffers from hypertension.

In some embodiments, the electronic device 200 stores m blood pressure models. m is an integer greater than 1. The electronic device 200 may input the feature value of the PPG signal into the m blood pressure models, to obtain m values of the hypertension risk of the user. The electronic device 200 combines the m values of the hypertension risk of the user, and determines the degree of the hypertension risk of the user. For example, the electronic device 200 may perform weighted averaging on the m values of the hypertension risk of the user. A larger result obtained through weighted averaging indicates a higher probability that systolic blood pressure of the user is higher than 140 mmHg and a higher risk that the user suffers from hypertension. A method for combining the m values of the hypertension risk is not limited in this embodiment of this application.

It can be learned from the hypertension risk detection method shown in FIG. 5 that when the user performs hypertension risk detection by using the electronic device 200 for the first time, a complex calibration operation may not be performed. The electronic device 200 may obtain, by using the PPG signal of the user, the blood pressure model that is adapted to the user, to detect the degree of the hypertension risk of the user. This can not only reduce impact of different physical conditions of different users on a detection result, to improve accuracy of hypertension risk detection, but also simplify a calibration operation performed by the user on the electronic device when the user detects the degree of the hypertension risk of the user, to improve convenience of performing hypertension risk detection by the user.

In some embodiments, the electronic device 200 may periodically or irregularly update the blood pressure model stored in the electronic device 200. Alternatively, in response to a user operation for updating the blood pressure model, the electronic device 200 may update the blood pressure model stored in the electronic device 200. The user operation for updating the blood pressure model may be, for example, the touch operation performed on the model update control 213 shown in FIG. 3A. An implementation process in which the electronic device 200 updates the blood pressure model stored in the electronic device 200 may be as follows: The electronic device 200 collects the PPG signal of the user, and sends the PPG signal of the user to the electronic device 100 to request to obtain the blood pressure model. The electronic device 100 may select, from the model library based on the received blood pressure model, one or more blood pressure models that are adapted to the user, and send the selected blood pressure model to the electronic device 200. The electronic device 200 may replace the stored blood pressure model with the received blood pressure model.

Compared with a calibration operation performed on a hypertension risk detection device by using a more professional hypertension risk detection device or by going to hospital for detection to obtain blood pressure with high accuracy, a method for updating, by the electronic device 200, the blood pressure model stored in the electronic device 200 in this application is simpler. The updated blood pressure model of the electronic device 200 is most adapted to a user in a current physical condition. This can not only reduce impact of a change of the physical condition of the user on the detection result, to improve accuracy of detecting the degree of the hypertension risk of the user, but also simplify a calibration operation performed by the user on the electronic device when the user detects the degree of the hypertension risk of the user, to improve convenience of performing hypertension risk detection by the user.

In some embodiments, a model establishment device may update the model library. Herein, an example in which the model establishment device is the electronic device 100 is used for description. Specifically, the electronic device 100 may add PPG signals in a PPG signal dataset and blood pressure corresponding to the PPG signals. A feature similarity between a feature of the added PPG signal and a feature of an original PPG signal in the PPG signal dataset is greater than a preset value. The electronic device 100 may establish a blood pressure model by using the added PPG signals and the blood pressure corresponding to the PPG signals, and add the blood pressure model to the model library. PPG signals for training the newly added blood pressure model in the model library and blood pressure corresponding to the PPG signals may be obtained from a cloud server.

In other words, the model library updated by the electronic device 100 may include more blood pressure models. The electronic device 100 may select an adapted blood pressure model for more users with different physical conditions by using the updated model library.

In some embodiments, a plurality of blood pressure models in the updated model library may include model attributes. A model attribute of one blood pressure model may be determined based on information about a user in one group of training data that is trained to obtain the blood pressure model and that is in the PPG signal dataset. The electronic device 100 may store the updated model library, a feature value of a PPG signal corresponding to each blood pressure model in the model library, and a model attribute of each blood pressure model in the model library. When selecting a blood pressure model that is adapted to one user, the electronic device 100 may select the blood pressure model from the updated model library by using a feature value of a PPG signal of the user and information about the user.

In some embodiments, the electronic device 200 may store the model library. The electronic device 200 may select, from the model library, the blood pressure model that is adapted to the user. The electronic device 200 may mark the selected blood pressure model. For example, the selected blood pressure model may correspondingly have a model identifier. An unselected blood pressure model does not have a model identifier. The model identifier may indicate that a blood pressure model corresponding to the model identifier is a blood pressure model that is adapted to the user. The electronic device 200 may detect the degree of the hypertension risk of the user by using the blood pressure model corresponding to the model identifier.

When updating a blood pressure model that is for detecting the degree of the hypertension risk of the user, the electronic device 200 may update the blood pressure model corresponding to the model identifier. For example, in the electronic device 200, a blood pressure model currently corresponding to the model identifier is a blood pressure model A. The electronic device 200 may collect the PPG signal of the user, and select a blood pressure model from the model library by using the PPG signal. The selected blood pressure model is a blood pressure model B. The electronic device 200 may remove a mark of the blood pressure model A, and mark the blood pressure model B. The blood pressure model corresponding to the model identifier is updated from the blood pressure model A to the blood pressure model B.

In the method shown in FIG. 5, step S102 may be that the electronic device 200 detects whether the blood pressure model in the model library correspondingly has a model identifier. If there is a blood pressure model correspondingly having a model identifier, the electronic device 200 may determine the degree of the hypertension risk of the user by using the blood pressure model corresponding to the model identifier. If there is no blood pressure model correspondingly having a model identifier, the electronic device 200 may select, from the model library by using the PPG signal of the user, the blood pressure model that is adapted to the user, and mark the selected blood pressure model. The marked blood pressure model may correspondingly have a model identifier. Further, the electronic device 200 may determine the degree of the hypertension risk of the user by using the blood pressure model corresponding to the model identifier.

**The following describes another communication system 20 provided in an embodiment of this application.**

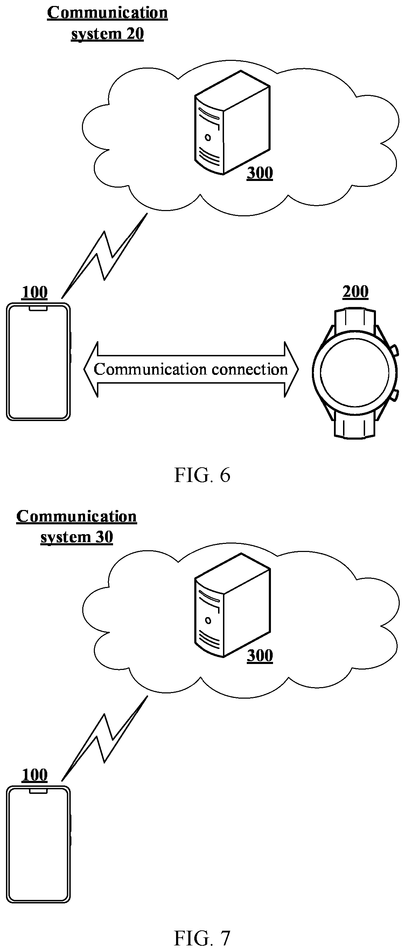

FIG. 6 is an architectural diagram of an example of a communication system 20. As shown in FIG. 2, the communication system 20 may include an electronic device 100, an electronic device 200, and a cloud server 300.

For the electronic device 100 and the electronic device 200, refer to the descriptions in the communication system 10. Details are not described herein again.

The electronic device 100 may be connected to the cloud server 300 by using a 2G network, a 3G network, a 4G network, a 5G network, a WLAN, or the like.

The cloud server 300 may store a PPG signal dataset. The cloud server 300 may train a blood pressure model by using the PPG signal dataset, and establish a model library. The cloud server 300 may further update the PPG signal dataset, and update the model library by using the updated signal dataset. In other words, the model establishment device in the foregoing embodiments may be the cloud server 300. For a method for training the blood pressure model and a method for updating the model library, refer to the descriptions in the foregoing embodiments. Details are not described herein again.

The cloud server 300 may send, to the electronic device 100, the model library and a feature value of a PPG signal corresponding to each blood pressure model in the model library. The electronic device 100 may store the received model library and the feature value of the PPG signal corresponding to each blood pressure model in the model library.

The electronic device 200 may be configured to: collect a PPG signal of a user, and detect a degree of a hypertension risk of the user by using a stored blood pressure model. The blood pressure model stored in the electronic device 200 is sent by the electronic device 100.

When the electronic device 200 updates the blood pressure model stored in the electronic device 200, the electronic device 200 may collect the PPG signal of the user, and send the collected PPG signal to the electronic device 100 to request to obtain a blood pressure model. The electronic device 100 may select, from the model library by using the received PPG signal, a blood pressure model that is adapted to the user to which the PPG signal belongs, and send the selected blood pressure model to the electronic device 200. The electronic device 200 may replace, with the received blood pressure model, the blood pressure model stored in the electronic device 200.

It should be noted that, in response to a user operation A that is performed on the electronic device 200 and that is for triggering the electronic device 200 to update the blood pressure model, the electronic device 200 may update the blood pressure model stored in the electronic device 200. The user operation A may be, for example, the touch operation performed on the model update control 213 shown in FIG. 3A. Optionally, in response to a user operation B that is performed on the electronic device 100 and that is for triggering the electronic device 100 to update the blood pressure model in the electronic device 200, the electronic device 100 may send an instruction for instructing the electronic device 200 to update the blood pressure model. When receiving the instruction, the electronic device 200 may update the blood pressure model stored in the electronic device 200. The user operation B may be, for example, the trigger operation performed on the model update control 233 shown in FIG. 3D. Optionally, the electronic device 200 may automatically periodically or irregularly update the blood pressure model stored in the electronic device 200.

In some embodiments, the cloud server 300 may alternatively send a model attribute of each blood pressure model in the model library to the electronic device 100. A model attribute of one blood pressure model may be determined based on information about a user in one group of training data that is trained to obtain the blood pressure model and that is in the PPG signal dataset. When selecting the blood pressure model that is adapted to the user, the electronic device 100 may select the blood pressure model from the model library in combination with information (for example, a gender, an age, a height, and a weight) of the user and the PPG signal of the user.

In the communication system 20 shown in FIG. 6, the cloud server 300 may establish and update the model library. Compared with the electronic device 100 and the electronic device 200, the cloud server 300 has higher operation and storage capabilities. This helps the electronic device 100 obtain a richer model library. A blood pressure model in the richer model library may be adapted to more users with different physical conditions. The electronic device 100 may select, from the model library, a blood pressure model that is more adapted to the user, to improve accuracy of detecting the degree of the hypertension risk of the user.

The electronic device 100 may select, from the model library, the blood pressure model that is adapted to the user. The electronic device 200 may determine the degree of the hypertension risk of the user by using the selected blood pressure model. This can reduce impact of different physical conditions of different users on a detection result. In addition, the electronic device 200 may update the blood pressure model for detecting the degree of the hypertension risk of the user. The updated blood pressure model is most adapted to a user in a current physical condition. This can not only reduce impact of a change of the physical condition of the user on the detection result, to improve accuracy of detecting the degree of the hypertension risk of the user, but also simplify a calibration operation performed by the user on the electronic device when the user detects the degree of the hypertension risk of the user, to improve convenience of performing hypertension risk detection by the user.

**The following describes another communication system 30 provided in an embodiment of this application.**

FIG. 7 is an architectural diagram of an example of a communication system 30. As shown in FIG. 7, the communication system 30 may include an electronic device 100 and a cloud server 300.

For a type and a structure of the electronic device 100, refer to the descriptions in the foregoing embodiments. The electronic device 100 may be connected to the cloud server 300 by using a 2G network, a 3G network, a 4G network, a 5G network, a WLAN, or the like.

The electronic device 100 may collect a PPG signal of a user. In a possible implementation, the electronic device 100 may collect the PPG signal of the user by using a camera and a flash. The electronic device 100 may use the flash as a light source. Light of the flash may be projected/reflected by a finger, a wrist, or another position of a person, and the returned light may be received by the camera. The electronic device 100 may determine the PPG signal of the user based on the light received by the camera. A method for collecting the PPG signal of the user by the electronic device 100 is not limited in this embodiment of this application. For details, refer to an implementation method for collecting a PPG signal by an electronic device, for example, a mobile phone or a tablet in a conventional technology.

The cloud server 300 may establish and update a model library. For a method for establishing and updating the model library by the cloud server 300, refer to the descriptions in the communication system 20. Details are not described herein again.

In some embodiments, the cloud server 200 may send, to the electronic device 100, the model library and a feature value of a PPG signal corresponding to each blood pressure model in the model library. The electronic device 100 may store the received model library and the feature value of the PPG signal corresponding to each blood pressure model in the model library.

The electronic device 100 may collect the PPG signal of the user, and select, from the model library by using the PPG signal, a blood pressure model that is adapted to the user. The electronic device 100 may mark the selected blood pressure model. For example, the selected blood pressure model may correspondingly have a model identifier. An unselected blood pressure model does not have a model identifier. The model identifier may indicate that a blood pressure model corresponding to the model identifier is a blood pressure model that is adapted to the user. The electronic device 100 may detect a degree of a hypertension risk of the user by using the blood pressure model corresponding to the model identifier.

When updating a blood pressure model that is for detecting the degree of the hypertension risk of the user, the electronic device 100 may update the blood pressure model corresponding to the model identifier. For a method for updating the blood pressure model corresponding to the model identifier by the electronic device 100, refer to the method for updating the blood pressure model corresponding to the model identifier by the electronic device 200 in the foregoing embodiments. Details are not described herein again.

In some embodiments, the cloud server 300 may alternatively send a model attribute of each blood pressure model in the model library to the electronic device 100. A model attribute of one blood pressure model may be determined based on information about a user in one group of training data that is trained to obtain the blood pressure model and that is in the PPG signal dataset. When selecting a blood pressure model that is adapted to the user, the electronic device 100 may select the blood pressure model from the model library in combination with information (for example, a gender, an age, a height, and a weight) of the user and the PPG signal of the user.

In some embodiments, the cloud server 300 may select, from the model library based on a PPG signal received by the user, a blood pressure model that is adapted to the user, and send the selected blood pressure model to the electronic device 100. The electronic device 100 may store the received blood pressure model, and detect a degree of a hypertension risk of the user by using the received blood pressure model.

Specifically, the electronic device 100 may collect the PPG signal of the user, and send the PPG signal of the user to the cloud server, to request to obtain a blood pressure model. The cloud server 300 may select the blood pressure model from the model library based on the received PPG signal, and send the selected blood pressure model to the electronic device 100.

If the electronic device 100 does not store a blood pressure model, the electronic device 100 may request, when detecting the degree of the hypertension risk of the user for the first time, to obtain the blood pressure model from the cloud server 300.

In response to a user operation for triggering the electronic device 100 to update the blood pressure model, the electronic device 100 may request to obtain the blood pressure model from the cloud server 300, and replace, with the blood pressure model obtained from the cloud server 300, the blood pressure model stored by the electronic device 100. The user operation may be, for example, the touch operation performed on the model update control 233 shown in FIG. 3D. Optionally, alternatively, the electronic device 100 may automatically periodically or irregularly update the blood pressure model stored in the electronic device 100.

In some embodiments, the cloud server 300 may select, from the model library in combination with the PPG signal of the user and information (for example, a gender, an age, a height, and a weight) about the user, a blood pressure model that is adapted to the user. The electronic device 100 may send the PPG signal of the user and the information about the user to the cloud server 300.

In the communication system 30 shown in FIG. 7, the electronic device 100 or the cloud server 300 may select, from the model library, the blood pressure model that is adapted to the user. The electronic device 100 may determine the degree of the hypertension risk of the user by using the selected blood pressure model. This can reduce impact of different physical conditions of different users on a detection result. In addition, the electronic device 100 may update the blood pressure model for detecting the degree of the hypertension risk of the user. The updated blood pressure model is most adapted to a user in a current physical condition. This can not only reduce impact of a change of the physical condition of the user on the detection result, to improve accuracy of detecting the degree of the hypertension risk of the user, but also simplify a calibration operation performed by the user on the electronic device when the user detects the degree of the hypertension risk of the user, to improve convenience of performing hypertension risk detection by the user.

**The following describes another communication system 40 provided in an embodiment of this application.**

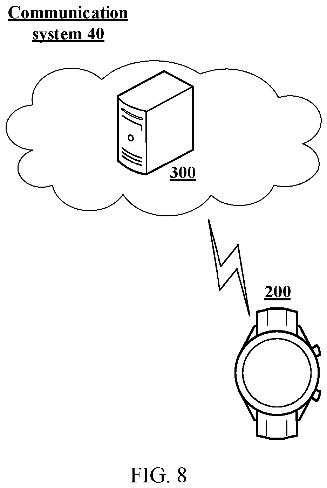

FIG. 8 is an architectural diagram of an example of a communication system 40. As shown in FIG. 8, the communication system 40 may include an electronic device 200 and a cloud server 300.

For a type and a structure of the electronic device 200, refer to the descriptions in the foregoing embodiments. The electronic device 200 may be connected to the cloud server 300 by using a 2G network, a 3G network, a 4G network, a 5G network, a WLAN, or the like.

The electronic device 200 may collect a PPG signal of a user, and detect a degree of a hypertension risk of the user by using a blood pressure model stored in the electronic device 200. The blood pressure model stored in the electronic device 200 is sent by the cloud server 300.

The cloud server 300 may establish and update a model library. For a method for establishing and updating the model library by the cloud server 300, refer to the descriptions in the communication system 20. Details are not described herein again.

In some embodiments, the cloud server 300 may select, from the model library based on a PPG signal received by the user, a blood pressure model that is adapted to the user, and send the selected blood pressure model to the electronic device 200. The electronic device 200 may store the received blood pressure model, and detect the degree of the hypertension risk of the user by using the received blood pressure model.

For a method in which the electronic device 200 requests to obtain a blood pressure model from the cloud server 300, refer to the method in which the electronic device 100 requests to obtain the blood pressure model from the cloud server in the foregoing embodiments. Details are not described herein again.

In the communication system 40 shown in FIG. 8, the electronic device 200 or the cloud server 300 may select, from the model library, the blood pressure model that is adapted to the user. The electronic device 200 may determine the degree of the hypertension risk of the user by using the selected blood pressure model. This can reduce impact of different physical conditions of different users on a detection result. In addition, the electronic device 200 may update a blood pressure model for detecting the degree of the hypertension risk of the user. The updated blood pressure model is most adapted to a user in a current physical condition. This can not only reduce impact of a change of the physical condition of the user on a detection result, to improve accuracy of detecting the degree of the hypertension risk of the user, but also simplify a calibration operation performed by the user on the electronic device when the user detects the degree of the hypertension risk of the user, to improve convenience of performing hypertension risk detection by the user.

In an embodiment of this application, an electronic device (for example, the electronic device 100 or the electronic device 200 in this application) may collect a first PPG signal of a first user. The first PPG signal may be collected by the electronic device when a degree of a hypertension risk is detected. For example, in response to a user operation performed on the measurement start control 212 shown in FIG. 3A, or in response to a user operation performed on the measurement start control 232 shown in FIG. 3D, the electronic device may collect a PPG signal to obtain the first PPG signal. The electronic device may determine the degree of the hypertension risk of the first user by using the first PPG signal and a blood pressure model.

The electronic device may further collect a second PPG signal of the first user. The second PPG signal may be collected when the electronic device selects a blood pressure model that is adapted to the first user. For example, when the electronic device automatically updates the blood pressure model periodically or irregularly, or in response to a user operation for updating the blood pressure model (for example, a user operation performed on a model update control 213 shown in FIG. 3A or a user operation performed on a model update control 233 shown in FIG. 3D), the electronic device may collect a PPG signal to obtain the second PPG signal. The electronic device may select, from a model library by using the second PPG signal, the blood pressure model that is adapted to the first user.

It should be noted that the first PPG signal and the second PPG signal may be a same PPG signal. For example, in response to the user operation performed on the measurement start control 212 shown in FIG. 3A, or in response to the user operation performed on the measurement start control 232 shown in FIG. 3D, the electronic device may collect the PPG signal to detect the degree of the hypertension risk of the first user. The electronic device has not yet determined the blood pressure model that is adapted to the first user. The electronic device may select, from the model library by using the collected PPG signal, the blood pressure model that is adapted to the first user. The electronic device may determine the degree of the hypertension risk of the first user by using the collected PPG signal and the selected blood pressure model.

In this embodiment of this application, one blood pressure model in the model library may be obtained by training one group of training samples. The group of training samples includes features of a plurality of PPG signals of one user and a degree of a hypertension risk of the user that is detected when the plurality of PPG signals are collected. The plurality of obtained PPG signals of the group of training samples are collected in a first time period. A time length of the first time period may be, for example, one week or one month. The time length of the first time period is not specifically limited in this embodiment of this application. In the first time period, a physical condition of a person usually does not change significantly. The plurality of PPG signals collected in the first time period may reflect a physical condition of the person in the first time period. The blood pressure model obtained through training by using the group of training samples can accurately detect a degree of a hypertension risk of a user in a corresponding physical condition.

When selecting the blood pressure model, the electronic device may compare a feature of a PPG signal of the user with the feature of the PPG signal of the group of training samples for training the blood pressure model, to determine whether a physical condition of the user corresponds to the blood pressure model.

In this embodiment of this application, a time interval between time for collecting the first PPG signal and time for collecting the second PPG signal does not exceed a time length of a second time period. The time length of the second time period may be, for example, one week or one month. The time length of the second time period is not specifically limited in this embodiment of this application. It can be learned from the foregoing embodiments that the first PPG signal may be for determining the degree of the hypertension risk of the user. The second PPG signal may be for selecting, from the model library, the blood pressure model that is adapted to the user. The electronic device may periodically or irregularly update the blood pressure model, or update the blood pressure model in response to the user operation for updating the blood pressure model. A time period in which the electronic device consecutively updates the blood pressure model twice is the second time period. In the time period between the two consecutive updates of the blood pressure model, the electronic device may detect the degree of the hypertension risk of the user by using a blood pressure model selected during a previous update of the blood pressure model.

In this embodiment of this application, the electronic device may receive a first user operation. The first user operation is a user operation indicating to select a blood pressure model. The first user operation may be, for example, a user operation performed on the model update control 213 shown in FIG. 3A, or a user operation performed on the model update control 233 shown in FIG. 3D.

In conclusion, the foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof. The modifications and replacements do not make the essence of the corresponding technical solutions depart from the scope of the technical solutions of embodiments of this application.

## Claims

1. A hypertension risk detection system, wherein the system comprises a first electronic device (200) and a second electronic device (100), wherein

   the second electronic device is configured to: select q blood pressure models from p blood pressure models for a first user, and send the q blood pressure models to the first electronic device, wherein the p blood pressure models are obtained by training p groups of training samples, one of the p blood pressure models is obtained by training one group of training samples, the group of training samples comprises features of a plurality of photoplethysmography, PPG signals of one user and a degree of a hypertension risk of the user that is detected when the PPG signal is collected, and the plurality of obtained PPG signals of the group of training samples are collected in a first time period, wherein p is a positive integer, and q is a positive integer less than or equal to p; and
   the first electronic device is configured to: collect a first PPG signal of the first user, and determine a degree of a hypertension risk of the first user by using the q blood pressure models and the first PPG signal;
   wherein the second electronic device is further configured to: obtain physiological feature information of the first user, and select w blood pressure models from the p blood pressure models by using the physiological feature information, wherein the physiological feature information comprises one or more of the following: an age, a gender, a height, and a weight, the w blood pressure models are obtained by training w groups of training samples in the p groups of training samples, and physiological feature information of a user to which PPG signals of the w groups of training samples belong and the physiological feature information of the first user fall within a same value range, wherein w is a positive integer less than or equal to p and greater than or equal to q;
   the first electronic device is further configured to: collect a second PPG signal of the first user, and send the second PPG signal to the second electronic device; and
   the second electronic device is further configured to select the q blood pressure models from the w blood pressure models by using the second PPG signal, wherein the q blood pressure models are obtained by training q groups of training samples in the w groups of training samples, and features of PPG signals of the q groups of training samples differ least from a feature of the second PPG signal in the w groups of training samples.

2. The system according to claim 1, wherein a time interval between time for collecting the first PPG signal and time for collecting the second PPG signal does not exceed a time length of a second time period.

3. The system according to claim 1 or 2, wherein before collecting the second PPG signal of the first user, the first electronic device (200) is further configured to receive a first user operation, wherein the first user operation indicates the first electronic device to collect the second PPG signal, and send the second PPG signal and a request for obtaining a blood pressure model to the second electronic device (100).

4. The system according to claim 1 or 2, wherein before collecting the second PPG signal of the first user, the first electronic device (200) is further configured to receive, from the second electronic device (100), a request for obtaining a PPG signal, wherein the request for obtaining the PPG signal is sent after the second electronic device receives a second user operation, and the second user operation indicates the second electronic device to select a blood pressure model.

5. An electronic device (200), wherein the electronic device comprises a PPG signal collection apparatus, a memory, and a processor, the PPG signal collection apparatus is configured to collect a PPG signal, the memory is configured to store a computer program, and the processor is configured to invoke the computer program, so that the electronic device performs the following steps:

obtaining (S101) a first photoplethysmography, PPG signal of a first user;

selecting (S103-S105) q blood pressure models from p blood pressure models for the first user, wherein the p blood pressure models are obtained by training p groups of training samples, one of the p blood pressure models is obtained by training one group of training samples, the group of training samples comprises features of a plurality of PPG signals of one user and a degree of a hypertension risk of the user that is detected when the PPG signal is collected, and the plurality of obtained PPG signals of the group of training samples are collected in a first time period, wherein p is a positive integer, and q is a positive integer less than or equal to p;

determining (S107) a degree of a hypertension risk of the first user by using the q blood pressure models and the first PPG signal;

wherein when selecting the q blood pressure models from the p blood pressure models for the first user, the electronic device specifically performs the following steps:

obtaining physiological feature information of the first user, wherein the physiological feature information comprises one or more of the following: an age, a gender, a height, and a weight;

selecting w blood pressure models from the p blood pressure models by using the physiological feature information of the first user, wherein the w blood pressure models are obtained by training w groups of training samples in the p groups of training samples, and physiological feature information of a user to which PPG signals of the w groups of training samples belong and the physiological feature information of the first user fall within a same value range, wherein w is a positive integer less than or equal to p and greater than or equal to q; and

obtaining a second PPG signal of the first user, and selecting the q blood pressure models from the w blood pressure models by using the second PPG signal, wherein the q blood pressure models are obtained by training q groups of training samples in the w groups of training samples, and features of PPG signals of the q groups of training samples differ least from a feature of the second PPG signal in the w groups of training samples.

6. The electronic device according to claim 5, wherein a time interval between time for collecting the first PPG signal and time for collecting the second PPG signal does not exceed a time length of a second time period.

7. The electronic device according to claim 5 or 6, wherein before obtaining the second PPG signal of the first user, the processor is configured to invoke the computer program, so that the electronic device (200) further performs the following steps:

receiving a first user operation, wherein the first user operation is a user operation indicating to select a blood pressure model.

8. The electronic device according to any one of claims 5 to 7, wherein a degree of a hypertension risk of a measured person that is detected when one PPG signal is collected is a probability that systolic blood pressure of the measured person is higher than 140 mmHg when the PPG signal is collected; or

a degree of a hypertension risk of a measured person that is detected when one PPG signal is collected is a probability that diastolic blood pressure of the measured person is higher than 90 mmHg when the PPG signal is collected.

9. A computer storage medium, comprising computer instructions, wherein when the computer instructions are run on an electronic device (200), the electronic device is enabled to perform the following steps:

obtaining (S101) a first photoplethysmography, PPG signal of a first user;

selecting (S103-S105) q blood pressure models from p blood pressure models for the first user, wherein the p blood pressure models are obtained by training p groups of training samples, one of the p blood pressure models is obtained by training one group of training samples, the group of training samples comprises features of a plurality of PPG signals of one user and a degree of a hypertension risk of the user that is detected when the PPG signal is collected, and the plurality of obtained PPG signals of the group of training samples are collected in a first time period, wherein p is a positive integer, and q is a positive integer less than or equal to p;

determining (S107) a degree of a hypertension risk of the first user by using the q blood pressure models and the first PPG signal;

wherein when selecting the q blood pressure models from the p blood pressure models for the first user, the electronic device specifically performs the following steps:

obtaining physiological feature information of the first user, wherein the physiological feature information comprises one or more of the following: an age, a gender, a height, and a weight;

selecting w blood pressure models from the p blood pressure models by using the physiological feature information of the first user, wherein the w blood pressure models are obtained by training w groups of training samples in the p groups of training samples, and physiological feature information of a user to which PPG signals of the w groups of training samples belong and the physiological feature information of the first user fall within a same value range, wherein w is a positive integer less than or equal to p and greater than or equal to q; and

obtaining a second PPG signal of the first user, and selecting the q blood pressure models from the w blood pressure models by using the second PPG signal, wherein the q blood pressure models are obtained by training q groups of training samples in the w groups of training samples, and features of PPG signals of the q groups of training samples differ least from a feature of the second PPG signal in the w groups of training samples.

**Patentansprüche**

1. Bluthochdruckrisiko-Detektionssystem, wobei das System eine erste elektronische Vorrichtung (200) und eine zweite elektronische Vorrichtung (100) umfasst, wobei die zweite elektronische Vorrichtung ausgelegt ist zum: Auswählen von q Blutdruckmodellen aus p Blutdruckmodellen für einen ersten Benutzer und Senden der q Blutdruckmodelle an die erste elektronische Vorrichtung, wobei die p Blutdruckmodelle erhalten werden durch Trainieren von p Gruppen von Trainingsstichproben, wobei eines der p Blutdruckmodelle erhalten wird durch Trainieren einer Gruppe von Trainingsstichproben, wobei die Gruppe von Trainingsstichproben Merkmale von mehreren Photoplethysmografie- bzw. PPG-Signalen eines Benutzers und einen Grad eines Bluthochdruckrisikos des Benutzers, der detektiert wird, wenn das PPG-Signal erfasst wird, umfasst, und wobei die mehreren erhaltenen PPG-Signale der Gruppe von Trainingsstichproben in einer ersten Zeitperiode erfasst werden, wobei p eine positive ganze Zahl ist und q eine positive ganze Zahl ist, die kleiner als oder gleich p ist; und

die erste elektronische Vorrichtung ausgelegt ist zum: Erfassen eines ersten PPG-Signals des ersten Benutzers und Bestimmen eines Grades eines Bluthochdruckrisikos des ersten Benutzers unter Verwendung der q Blutdruckmodelle und des ersten PPG-Signals;

wobei die zweite elektronische Vorrichtung ferner ausgelegt ist zum: Erhalten von physiologischen Merkmalsinformationen des ersten Benutzers und Auswählen von w Blutdruckmodellen aus den p Blutdruckmodellen unter Verwendung der physiologischen Merkmalsinformationen, wobei die physiologischen Merkmalsinformationen eines oder mehrere aus Folgendem umfassen: ein Alter, ein Geschlecht, eine Größe und ein Gewicht, wobei die w Blutdruckmodelle erhalten werden durch Trainieren von w Gruppen von Trainingsstichproben in den p Gruppen von Trainingsstichproben, und wobei physiologische Merkmalsinformationen eines Benutzers, zu dem PPG-Signale der w Gruppen von Trainingsstichproben gehören, und die physiologischen Merkmalsinformationen des ersten Benutzers in einen gleichen Wertebereich fallen, wobei w eine positive ganze Zahl ist, die kleiner als oder gleich p ist und größer als oder gleich q ist;

die erste elektronische Vorrichtung ferner ausgelegt ist zum: Erfassen eines zweiten PPG-Signals des ersten Benutzers und Senden des zweiten PPG-Signals an die zweite elektronische Vorrichtung; und

die zweite elektronische Vorrichtung ferner ausgelegt ist zum Auswählen der q Blutdruckmodelle aus den w Blutdruckmodellen unter Verwendung des zweiten PPG-Signals, wobei die q Blutdruckmodelle erhalten werden durch Trainieren von q Gruppen von Trainingsstichproben in den w Gruppen von Trainingsstichproben, und wobei sich Merkmale von PPG-Signalen der q Gruppen von Trainingsstichproben am wenigsten von einem Merkmal des zweiten PPG-Signals in den w Gruppen von Trainingsstichproben unterscheiden.

2. System nach Anspruch 1, wobei ein Zeitintervall zwischen der Zeit zum Erfassen des ersten PPG-Signals und der Zeit zum Erfassen des zweiten PPG-Signals eine Zeitlänge einer zweiten Zeitperiode nicht überschreitet.

3. System nach Anspruch 1 oder 2, wobei, vor dem Erfassen des zweiten PPG-Signals des ersten Benutzers, die erste elektronische Vorrichtung (200) ferner ausgelegt ist zum Empfangen einer ersten Benutzeroperation, wobei die erste Benutzeroperation der ersten elektronischen Vorrichtung anzeigt, das zweite PPG-Signal zu erfassen und das zweite PPG-Signal sowie eine Anforderung zum Erhalten eines Blutdruckmodells an die zweite elektronische Vorrichtung (100) zu senden.

4. System nach Anspruch 1 oder 2, wobei, vor dem Erfassen des zweiten PPG-Signals des ersten Benutzers, die erste elektronische Vorrichtung (200) ferner ausgelegt ist zum Empfangen, von der zweiten elektronischen Vorrichtung (100), einer Anforderung zum Erhalten eines PPG-Signals, wobei die Anforderung zum Erhalten des PPG-Signals gesendet wird, nachdem die zweite elektronische Vorrichtung eine zweite Benutzeroperation empfängt, und wobei die zweite Benutzeroperation der zweiten elektronischen Vorrichtung anzeigt, ein Blutdruckmodell auszuwählen.

5. Elektronische Vorrichtung (200), wobei die elektronische Vorrichtung eine PPG-Signalerfassungseinrichtung, einen Speicher und einen Prozessor umfasst, wobei die PPG-Signalerfassungseinrichtung ausgelegt ist zum Erfassen eines PPG-Signals, der Speicher ausgelegt ist zum Speichern eines Computerprogramms und der Prozessor ausgelegt ist zum Aufrufen des Computerprogramms, sodass die elektronische Vorrichtung die folgenden Schritte durchführt:

Erhalten (S101) eines ersten Photoplethysmografie- bzw. PPG-Signals eines ersten Benutzers;
Auswählen (S103-S105) von q Blutdruckmodellen aus p Blutdruckmodellen für den ersten Benutzer, wobei die p Blutdruckmodelle erhalten werden durch Trainieren von p Gruppen von Trainingsstichproben, wobei eines der p Blutdruckmodelle erhalten wird durch Trainieren einer Gruppe von Trainingsstichproben, wobei die Gruppe von Trainingsstichproben Merkmale von mehreren PPG-Signalen eines Benutzers und einen Grad eines Bluthochdruckrisikos des Benutzers, der detektiert wird, wenn das PPG-Signal erfasst wird, umfasst, und wobei die mehreren erhaltenen PPG-Signale der Gruppe von Trainingsstichproben in einer ersten Zeitperiode erfasst werden, wobei p eine positive ganze Zahl ist und q eine positive ganze Zahl ist, die kleiner als oder gleich p ist;
Bestimmen (S107) eines Grades eines Bluthochdruckrisikos des ersten Benutzers unter Verwendung der q Blutdruckmodelle und des ersten PPG-Signals;
wobei, beim Auswählen der q Blutdruckmodelle aus den p Blutdruckmodellen für den ersten Benutzer, die elektronische Vorrichtung speziell die folgenden Schritte durchführt: Erhalten von physiologischen Merkmalsinformationen des ersten Benutzers, wobei die physiologischen Merkmalsinformationen eines oder mehrere aus Folgendem umfassen: ein Alter, ein Geschlecht, eine Größe und ein Gewicht;
Auswählen von w Blutdruckmodellen aus den p Blutdruckmodellen unter Verwendung der physiologischen Merkmalsinformationen des ersten Benutzers, wobei die w Blutdruckmodelle erhalten werden durch Trainieren von w Gruppen von Trainingsstichproben in den p Gruppen von Trainingsstichproben, und wobei physiologische Merkmalsinformationen eines Benutzers, zu dem PPG-Signale der w Gruppen von Trainingsstichproben gehören, und die physiologischen Merkmalsinformationen des ersten Benutzers in einen gleichen Wertebereich fallen, wobei w eine positive ganze Zahl ist, die kleiner als oder gleich p ist und größer als oder gleich q ist; und
Erhalten eines zweiten PPG-Signals des ersten Benutzers und Auswählen der q Blutdruckmodelle aus den w Blutdruckmodellen unter Verwendung des zweiten PPG-Signals, wobei die q Blutdruckmodelle erhalten werden durch Trainieren von q Gruppen von Trainingsstichproben in den w Gruppen von Trainingsstichproben, und wobei sich Merkmale von PPG-Signalen der q Gruppen von Trainingsstichproben am wenigsten von einem Merkmal des zweiten PPG-Signals in den w Gruppen von Trainingsstichproben unterscheiden.

6. Elektronische Vorrichtung nach Anspruch 5, wobei ein Zeitintervall zwischen der Zeit zum Erfassen des ersten PPG-Signals und der Zeit zum Erfassen des zweiten PPG-Signals eine Zeitlänge einer zweiten Zeitperiode nicht überschreitet.

7. Elektronische Vorrichtung nach Anspruch 5 oder 6, wobei, vor dem Erhalten des zweiten PPG-Signals des ersten Benutzers, der Prozessor ausgelegt ist zum Aufrufen des Computerprogramms, sodass die elektronische Vorrichtung (200) ferner die folgenden Schritte durchführt:
Empfangen einer ersten Benutzeroperation, wobei die erste Benutzeroperation eine Benutzeroperation ist, die anzeigt, ein Blutdruckmodell auszuwählen.

8. Elektronische Vorrichtung nach einem der Ansprüche 5 bis 7, wobei ein Grad eines Bluthochdruckrisikos einer gemessenen Person, der detektiert wird, wenn ein PPG-Signal erfasst wird, eine Wahrscheinlichkeit ist, dass systolischer Blutdruck der gemessenen Person höher als 140 mmHg ist, wenn das PPG-Signal erfasst wird; oder ein Grad eines Bluthochdruckrisikos einer gemessenen Person, der detektiert wird, wenn ein PPG-Signal erfasst wird, eine Wahrscheinlichkeit ist, dass diastolischer Blutdruck der gemessenen Person höher als 90 mmHg ist, wenn das PPG-Signal erfasst wird.

9. Computerspeichermedium, umfassend Computeranweisungen, wobei, wenn die Computeranweisungen auf einer elektronischen Vorrichtung (200) ausgeführt werden, die elektronische Vorrichtung in die Lage versetzt wird, die folgenden Schritte durchzuführen:

Erhalten (S101) eines ersten Photoplethysmografie- bzw. PPG-Signals eines ersten Benutzers;
Auswählen (S103-S105) von q Blutdruckmodellen aus p Blutdruckmodellen für den ersten Benutzer, wobei die p Blutdruckmodelle erhalten werden durch Trainieren von p Gruppen von Trainingsstichproben, wobei eines der p Blutdruckmodelle erhalten wird durch Trainieren einer Gruppe von Trainingsstichproben, wobei die Gruppe von Trainingsstichproben Merkmale von mehreren PPG-Signalen eines Benutzers und einen Grad eines Bluthochdruckrisikos des Benutzers, der detektiert wird, wenn das PPG-Signal erfasst wird, umfasst, und wobei die mehreren erhaltenen PPG-Signale der Gruppe von Trainingsstichproben in einer ersten Zeitperiode erfasst werden, wobei p eine positive ganze Zahl ist und q eine positive ganze Zahl ist, die kleiner als oder gleich p ist;
Bestimmen (S107) eines Grades eines Bluthochdruckrisikos des ersten Benutzers unter Verwendung der q Blutdruckmodelle und des ersten PPG-Signals;
wobei, beim Auswählen der q Blutdruckmodelle aus den p Blutdruckmodellen für den ersten Benutzer, die elektronische Vorrichtung speziell die folgenden Schritte durchführt: Erhalten von physiologischen Merkmalsinformationen des ersten Benutzers, wobei die physiologischen Merkmalsinformationen eines oder mehrere aus Folgendem umfassen: ein Alter, ein Geschlecht, eine Größe und ein Gewicht;
Auswählen von w Blutdruckmodellen aus den p Blutdruckmodellen unter Verwendung der physiologischen Merkmalsinformationen des ersten Benutzers, wobei die w Blutdruckmodelle erhalten werden durch Trainieren von w Gruppen von Trainingsstichproben in den p Gruppen von Trainingsstichproben, und wobei physiologische Merkmalsinformationen eines Benutzers, zu dem PPG-Signale der w Gruppen von Trainingsstichproben gehören, und die physiologischen Merkmalsinformationen des ersten Benutzers in einen gleichen Wertebereich fallen, wobei w eine positive ganze Zahl ist, die kleiner als oder gleich p ist und größer als oder gleich q ist; und
Erhalten eines zweiten PPG-Signals des ersten Benutzers und Auswählen der q Blutdruckmodelle aus den w Blutdruckmodellen unter Verwendung des zweiten PPG-Signals, wobei die q Blutdruckmodelle erhalten werden durch Trainieren von q Gruppen von Trainingsstichproben in den w Gruppen von Trainingsstichproben, und wobei sich Merkmale von PPG-Signalen der q Gruppen von Trainingsstichproben am wenigsten von einem Merkmal des zweiten PPG-Signals in den w Gruppen von Trainingsstichproben unterscheiden.

## Revendications

1. Système de détection de risque d'hypertension, lequel système comprend un premier dispositif électronique (200) et un deuxième dispositif électronique (100),

le deuxième dispositif électronique étant configuré pour : sélectionner q modèles de pression sanguine parmi p modèles de pression sanguine pour un premier utilisateur, et envoyer les q modèles de pression sanguine au premier dispositif électronique, les p modèles de pression sanguine étant obtenus par entraînement de p groupes d'échantillons d'apprentissage, l'un des p modèles de pression sanguine étant obtenu par entraînement d'un groupe d'échantillons d'apprentissage, le groupe d'échantillons d'apprentissage comprenant des caractéristiques d'une pluralité de signaux de photopléthysmographie, PPG, d'un utilisateur et un degré de risque d'hypertension de l'utilisateur qui est détecté lorsque le signal PPG est collecté, et la pluralité de signaux PPG obtenus du groupe d'échantillons d'apprentissage étant collectés dans une première période de temps, p étant un entier positif et q étant un entier positif inférieur ou égal à p ; et
le premier dispositif électronique étant configuré pour : collecter un premier signal PPG du premier utilisateur, et déterminer un degré de risque d'hypertension du premier utilisateur en utilisant les q modèles de pression sanguine et le premier signal PPG ;
le deuxième dispositif électronique étant en outre configuré pour : obtenir des informations de caractéristiques physiologiques du premier utilisateur, et sélectionner w modèles de pression sanguine parmi les p modèles de pression sanguine en utilisant les informations de caractéristiques physiologiques, les informations de caractéristiques physiologiques comprenant une ou plusieurs informations parmi les suivantes : un âge, un genre, une taille et un poids, les w modèles de pression sanguine étant obtenus par entraînement de w groupes d'échantillons d'apprentissage parmi les p groupes d'échantillons d'apprentissage, et des informations de caractéristiques physiologiques d'un utilisateur auquel les signaux PPG des w groupes d'échantillons d'apprentissage appartiennent et les informations de caractéristiques physiologiques du premier utilisateur tombant dans une même plage de valeurs, w étant un entier positif inférieur ou égal à p et supérieur ou égal à q ;
le premier dispositif électronique étant en outre configuré pour : collecter un deuxième signal PPG du premier utilisateur, et envoyer le deuxième signal PPG au deuxième dispositif électronique ; et

le deuxième dispositif électronique étant en outre configuré pour sélectionner les q modèles de pression sanguine parmi les w modèles de pression sanguine en utilisant le deuxième signal PPG, les q modèles de pression sanguine étant obtenus par entraînement de q groupes d'échantillons d'apprentissage parmi les w groupes d'échantillons d'apprentissage, et les caractéristiques des signaux PPG des q groupes d'échantillons d'apprentissage étant celles qui diffèrent le moins d'une caractéristique du deuxième signal PPG dans les w groupes d'échantillons d'apprentissage.

2. Système selon la revendication 1, dans lequel un intervalle de temps entre le moment de collecte du premier signal PPG et le moment de collecte du deuxième signal PPG n'excède pas une durée d'une deuxième période de temps.

3. Système selon la revendication 1 ou 2, dans lequel, avant la collecte du deuxième signal PPG du premier utilisateur, le premier dispositif électronique (200) est en outre configuré pour recevoir une première opération d'utilisateur, la première opération d'utilisateur indiquant au premier dispositif électronique de collecter le deuxième signal PPG, et pour envoyer le deuxième signal PPG et une demande d'obtention d'un modèle de pression sanguine au deuxième dispositif électronique (100).

4. Système selon la revendication 1 ou 2, dans lequel, avant la collecte du deuxième signal PPG du premier utilisateur, le premier dispositif électronique (200) est en outre configuré pour recevoir, en provenance du deuxième dispositif électronique (100), une demande d'obtention d'un signal PPG, la demande d'obtention du signal PPG étant envoyée après que le deuxième dispositif électronique a reçu une deuxième opération d'utilisateur, et la deuxième opération d'utilisateur indiquant au deuxième dispositif électronique de sélectionner un modèle de pression sanguine.

5. Dispositif électronique (200), dans lequel le dispositif électronique comprend un appareil de collecte de signal PPG, une mémoire et un processeur, l'appareil de collecte de signal PPG étant configuré pour collecter un signal PPG, la mémoire étant configurée pour stocker un programme informatique, et le processeur étant configuré pour invoquer le programme informatique, de telle sorte que le dispositif électronique exécute les étapes suivantes :

l'obtention (S101) d'un premier signal de photopléthysmographie, PPG, d'un premier utilisateur ;

la sélection (S103-S105) de q modèles de pression sanguine parmi les p modèles de pression sanguine pour le premier utilisateur, les p modèles de pression sanguine étant obtenus par entraînement de p groupes d'échantillons d'apprentissage, l'un des p modèles de pression sanguine étant obtenu par entraînement d'un groupe d'échantillons d'apprentissage, le groupe d'échantillons d'apprentissage comprenant des caractéristiques d'une pluralité de signaux PPG d'un utilisateur et un degré de risque d'hypertension de l'utilisateur qui est détecté lorsque le signal PPG est collecté, et la pluralité de signaux PPG obtenus du groupe d'échantillons d'apprentissage étant collectés dans une première période de temps, p étant un entier positif et q étant un entier positif inférieur ou égal à p ;

la détermination (S107) d'un degré de risque d'hypertension du premier utilisateur en utilisant les q modèles de pression sanguine et le premier signal PPG ;

le dispositif électronique, lors de la sélection des q modèles de pression sanguine parmi les p modèles de pression sanguine pour le premier utilisateur, exécutant spécifiquement les étapes suivantes :

l'obtention d'informations de caractéristiques physiologiques du premier utilisateur, les informations de caractéristiques physiologiques comprenant une ou plusieurs informations parmi les suivantes : un âge, un genre, une taille et un poids ;

la sélection de w modèles de pression sanguine parmi les p modèles de pression sanguine en utilisant les informations de caractéristiques physiologiques du premier utilisateur, les w modèles de pression sanguine étant obtenus par entraînement de w groupes d'échantillons d'apprentissage dans les p groupes d'échantillons d'apprentissage, et des informations de caractéristiques physiologiques d'un utilisateur auquel les signaux PPG des w groupes d'échantillons d'apprentissage appartiennent et les informations de caractéristiques physiologiques du premier utilisateur tombant dans une même plage de valeurs, w étant un entier positif inférieur ou égal à p et supérieur ou égal à q ; et

l'obtention d'un deuxième signal PPG du premier utilisateur, et la sélection des q modèles de pression sanguine parmi les w modèles de pression sanguine en utilisant le deuxième signal PPG, les q modèles de pression sanguine étant obtenus par entraînement de q groupes d'échantillons d'apprentissage parmi les w groupes d'échantillons d'apprentissage, et les caractéristiques des signaux PPG des q groupes d'échantillons d'apprentissage étant celles qui diffèrent le moins d'une caractéristique du deuxième signal PPG dans les w groupes d'échantillons d'apprentissage.

**6.** Dispositif électronique selon la revendication 5, dans lequel un intervalle de temps entre le moment de collecte du premier signal PPG et le moment de collecte du deuxième signal PPG n'excède pas une durée d'une deuxième période de temps.

**7.** Dispositif électronique selon la revendication 5 ou 6, dans lequel, avant l'obtention du deuxième signal PPG du premier utilisateur, le processeur est configuré pour invoquer le programme informatique, de façon que le dispositif électronique (200) exécute en outre l'étape suivante :
la réception d'une première opération d'utilisateur, la première opération d'utilisateur étant une opération d'utilisateur indiquant de sélectionner un modèle de pression sanguine.

**8.** Dispositif électronique selon l'une quelconque des revendications 5 à 7, dans lequel un degré de risque d'hypertension d'une personne mesurée qui est détecté lorsqu'un signal PPG est collecté est une probabilité que la pression sanguine systolique de la personne mesurée soit supérieure à 140 mmHg lorsque le signal PPG est collecté ; ou un degré de risque d'hypertension d'une personne mesurée qui est détecté lorsqu'un signal PPG est collecté est une probabilité que la pression sanguine diastolique de la personne mesurée soit supérieure à 90 mmHg lorsque le signal PPG est collecté.

**9.** Support de stockage informatique, comprenant des instructions informatiques, dans lequel, lorsque les instructions informatiques sont exécutées sur un dispositif électronique (200), le dispositif électronique est autorisé à exécuter les étapes suivantes :

l'obtention (S101) d'un premier signal de photopléthysmographie, PPG, d'un premier utilisateur ;
la sélection (S103-S105) de q modèles de pression sanguine parmi les p modèles de pression sanguine pour le premier utilisateur, les p modèles de pression sanguine étant obtenus par entraînement de p groupes d'échantillons d'apprentissage, l'un des p modèles de pression sanguine étant obtenu par entraînement d'un groupe d'échantillons d'apprentissage, le groupe d'échantillons d'apprentissage comprenant des caractéristiques d'une pluralité de signaux PPG d'un utilisateur et un degré de risque d'hypertension de l'utilisateur qui est détecté lorsque le signal PPG est collecté, et la pluralité de signaux PPG obtenus du groupe d'échantillons d'apprentissage étant collectés dans une première période de temps, p étant un entier positif et q étant un entier positif inférieur ou égal à p ;
la détermination (S107) d'un degré de risque d'hypertension du premier utilisateur en utilisant les q modèles de pression sanguine et le premier signal PPG ;
le dispositif électronique, lors de la sélection des q modèles de pression sanguine parmi les p modèles de pression sanguine pour le premier utilisateur, exécutant spécifiquement les étapes suivantes :

l'obtention d'informations de caractéristiques physiologiques du premier utilisateur, les informations de caractéristiques physiologiques comprenant une ou plusieurs informations parmi les suivantes : un âge, un genre, une taille et un poids ;
la sélection de w modèles de pression sanguine parmi les p modèles de pression sanguine en utilisant les informations de caractéristiques physiologiques du premier utilisateur, les w modèles de pression sanguine étant obtenus par entraînement de w groupes d'échantillons d'apprentissage dans les p groupes d'échantillons d'apprentissage, et des informations de caractéristiques physiologiques d'un utilisateur auquel les signaux PPG des w groupes d'échantillons d'apprentissage appartiennent et les informations de caractéristiques physiologiques du premier utilisateur tombant dans une même plage de valeurs, w étant un entier positif inférieur ou égal à p et supérieur ou égal à q ; et
l'obtention d'un deuxième signal PPG du premier utilisateur, et la sélection des q modèles de pression sanguine parmi les w modèles de pression sanguine en utilisant le deuxième signal PPG, les q modèles de pression sanguine étant obtenus par entraînement de q groupes d'échantillons d'apprentissage parmi les w groupes d'échantillons d'apprentissage, et les caractéristiques des signaux PPG des q groupes d'échantillons d'apprentissage étant celles qui diffèrent le moins d'une caractéristique du deuxième signal PPG dans les w groupes d'échantillons d'apprentissage.

**Communication system 10**

**100**

**200**

Communication connection

FIG. 1

Electronic device 100

Antenna 1        Antenna 2

| Mobile communications module 2G/3G/4G/5G [150] | Wireless communications module BT/WLAN/GNSS/NFC/IR/FM [160] |

Speaker [170A]

Receiver [170B]

Microphone [170C]

Headset jack [170D]

Audio module [170]

Displays 1 to N [194]

Cameras 1 to N [193]

Indicator [192]

Motor [191]

Button [190]

Internal memory [121]

SIM card interfaces 1 to N [195]

External memory interface [120]

Processor [110]

Sensor module [180]

Pressure sensor [180A]

Gyro sensor [180B]

Barometric pressure sensor [180C]

Magnetic sensor [180D]

Acceleration sensor [180E]

Distance sensor [180F]

Optical proximity sensor [180G]

Fingerprint sensor [180H]

Temperature sensor [180J]

Touch sensor [180K]

Ambient light sensor [180L]

Bone conduction sensor [180M]

USB interface [130]

Charging input

Charging management module [140]

Power management module [141]

Battery [142]

FIG. 2

FIG. 3A

FIG. 3B

Hypertension risk level: high

According to your blood pressure level in the past month, it is preliminarily determined that you may belong to a hypertensive group. Please contact a professional doctor for diagnosis in time.

FIG. 3C

100

5G

8:00

230

← Hypertension risk screening

Nickname:     Xiao Ming

231

Age:          20

Gender:       Male

Height:       180 cm

Weight:       70 kg

**Start measurement**     232

233

Check and update of a screening model*

*The updated screening model can more accurately measure your current hypertension risk

FIG. 3D

100

240

5G

8:00

← Screening result

Hypertension risk trend

| Day | Week | Month |

High

Medium

Low

Normal

7:35 8:00 11:20 15:00 15:30 20:03 22:12 22:29 23:32

Measured nine times a day, including two
times of high blood pressure

FIG. 3E

100

← Screening result

250

Hypertension risk level: high

According to your blood pressure level in the past month, it is preliminarily determined that you may belong to a hypertensive group. Please contact a professional doctor for diagnosis in time.

FIG. 3F

FIG. 4A

FIG. 4B

Electronic device 100
(storing a model library,
where the model library
includes a plurality of blood
pressure models)

Electronic device
200

S101. Collect a PPG signal
of a user

S102. Detect
whether a blood pressure
model is stored

No

S103. Send the PPG signal of the user, and request
to obtain a blood pressure model

Yes

S104. Select the blood pressure
model from the model library
based on the received PPG signal

S105. Send the selected blood pressure model

S106. Store the
received blood
pressure model

S107. Determine a degree of a
hypertension risk of the user
based on the PPG signal of the
user by using the stored blood
pressure model

FIG. 5

**Communication
system 20**

Communication connection

FIG. 6

**Communication
system 30**

FIG. 7

**Communication
system 40**

**300**

**200**

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020073012 A1